# EUROPÄISCHE PATENTSCHRIFT

(11) **EP 3 864 121 B1**
(45) Veröffentlichungstag und Bekanntmachung des Hinweises auf die Patenterteilung: **29.11.2023**
(21) Anmeldenummer: 19732320.7
(22) Anmeldetag: 18.06.2019
(51) Int. Cl.: C11D 3/16, C11D 3/40, C07F 5/00, C07F 13/00, C07F 15/02, C11D 17/04

(54) **WASCHMITTELZUSAMMENSETZUNG MIT CATECHOL-METALLKOMPLEXVERBINDUNG**
LAUNDRY DETERGENT COMPOSITION COMPRISING A CATECHOL METAL COMPLEX COMPOUND
COMPOSITION DÉTERGENTE COMPRENANT UN COMPOSÉ DE COMPLEXE MÉTALLIQUE DE CATÉCHOL

(30) Priorität: 11.10.2018 DE 102018217393
(43) Veröffentlichungstag der Anmeldung: 18.08.2021
(73) Patentinhaber: Henkel AG & Co. KGaA, 40589 Düsseldorf (DE)
(72) Erfinder: MEIER, Frank, 40589 Düsseldorf (DE); JOB, Mareile, 51375 Leverkusen (DE); KROPF, Christian, 40724 Hilden (DE); PEGELOW, Ulrich, 40597 Düsseldorf (DE)
(86) Internationale Anmeldenummer: PCT/EP2019/065984
(87) Internationale Veröffentlichungsnummer: WO 2020/074142

(56) Entgegenhaltungen:
- DE-A1-102016 214 660
- US-A1- 2009 176 684
- TIMOTHY B. KARPISHIN ET AL: "Stereoselectivity in chiral iron(III) and gallium(III) tris(catecholate) complexes effected by nonbonded weakly polar interactions", JOURNAL OF THE AMERICAN CHEMICAL SOCIETY, Bd. 115, Nr. 14, 1. Juli 1993 (1993-07-01) , Seiten 6115-6125, XP055037767, ISSN: 0002-7863, DOI: 10.1021/ja00067a029
- K.M. CLARKE JURCHEN ET AL.: "A Bidentate Terephthalamide Ligand, TAMmeg, as an Entry into Terephthalamide-Containing Therapeutic Iron Chelating Agents", INORGANIC CHEMISTRY, Bd. 45, Nr. 6, 18. Februar 2006 (2006-02-18), Seiten 2438-2446, XP002794654,
- HANG YIN ET AL: "Terephthalamide Derivatives as Mimetics of Helical Peptides:? Disruption of the Bcl-x L /Bak Interaction", JOURNAL OF THE AMERICAN CHEMICAL SOCIETY, Bd. 127, Nr. 15, 1. April 2005 (2005-04-01), Seiten 5463-5468, XP055057768, ISSN: 0002-7863, DOI: 10.1021/ja0446404

## Beschreibung

Die vorliegende Erfindung liegt auf dem technischen Gebiet von Waschmittelzusammensetzungen, insbesondere für Textilien, und betrifft insbesondere die Einfärbung von Waschmitteln. Ferner betrifft die vorliegende Erfindung spezielle Catechol-Metall-Komplexverbindungen, deren Herstellung sowie ein Verfahren zur Textilbehandlung mit besagter Waschmittelzusammensetzung.

Die Ästhetik spielt bei Flüssigwaschmitteln, insbesondere in durchsichtigen oder transluzenten Flaschen oder bei vorportionierten Produkten wie Pouches, eine wichtige Rolle. Insbesondere Produkte mit intensiven und kräftigen Farben sind vom Verbraucher gewünscht. Ein Risiko bei der Verwendung neuer Farbstoffe und/oder hoher Einsatzmengen von Farbstoffen besteht darin, dass diese Farbstoffe während des Waschprozesses auf das Textil aufziehen und es zu einer lokalen oder einheitlichen Verfärbung kommt. Der Effekt ist in Labortests mit der "Anfärbeneigung" messbar und ist auch für den Konsumenten visuell wahrnehmbar. Das gewaschene Kleidungsstück ist unter Umständen irreversibel geschädigt und die Verfärbung lässt sich auch mit erneutem Waschen nicht mehr entfernen.

Die Aufgabe der vorliegenden Erfindung war es deshalb ein Waschmittel bereitzustellen, dass bei Lagerung eine intensive Farbe aufweist und farbstabil ist, jedoch das Textil im Waschprozess nicht verfärbt.

Die Erfinder der vorliegenden Erfindung haben überraschend gefunden, dass diese Aufgabe durch die Verwendung spezieller Catechol-Metallkomplexverbindungen als Farbstoff möglich ist. Die Verwendung spezieller Catechol-Metallkomplexverbindungen reduziert das Risiko der unerwünschten Textilanfärbung, da besagte Komplexverbindungen eine sehr geringe Affinität für natürliche und synthetische Gewebe aufweisen.

Zudem zeigen Waschmittel mit den erfindungsgemäßen Catechol-Metallkomplexverbindungen in Gegenwart eines Überschusses an freiem Catecholliganden eine gute Bleichwirkung. Die Waschmittel sind besonders vorteilhaft in ihrer Reinigungswirkung gegenüber Anschmutzungen, die nur durch Bleichen entfernt werden können. Bei diesen Anschmutzungen handelt es sich um solche, die polymerisierbare Substanzen enthalten. Bei den polymerisierbaren Substanzen handelt es sich vor allem um polyphenolische Farbstoffe, vorzugsweise um Flavonoide, insbesondere aus der Klasse der Anthocyanidine oder Anthocyane. Die Anschmutzungen können insbesondere durch Lebensmittelprodukte oder Getränke verursacht worden sein, die entsprechende Farbstoffe enthalten. Bei den Anschmutzungen kann es sich insbesondere um Flecken von Früchten oder Gemüse oder auch Rotweinflecken handeln, die insbesondere polyphenolische Farbstoffe, vor allem solche aus der Klasse der Anthocyanidine oder Anthocyane, enthalten. US 2009/176684 A1 offenbart Waschmittel, enthaltend 1,2-Dihydroxybenzol-3,5-disulfonsäure. DE102016214660 A 1beschreibt Waschmittel, enthaltend Dihydroxyterephthalsäurederivate.

Offenbart ist eine Catechol-Metallkomplexverbindung der Formel (I) 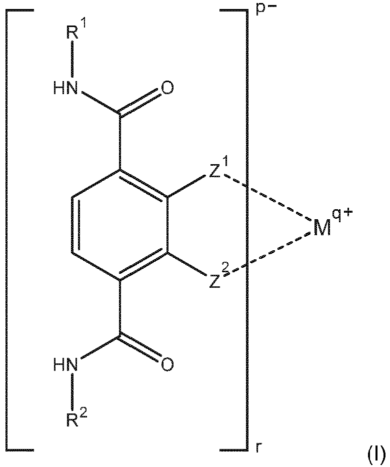 wobei
- R¹ und R²: unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X⁻, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen,
- Z¹ und Z²: unabhängig voneinander für OH oder O⁻ stehen,
- M: steht für ein Metallkation eines Übergangsmetalls oder Lanthanoids,
- q: als Ladungszahl des Metallkations M für eine Zahl 2, 3 oder 4 steht,
- p: als Ladungszahl des Catecholliganden für eine Zahl 0, 1 oder 2 steht,
- r: für eine Zahl 1, 2, 3 oder 4 steht.

"Flüssig", wie hierin in Bezug auf erfindungsgemäße Waschmittel verwendet, schließt alle bei Standardbedingungen (20 °C, 1013 mbar) fließfähigen Waschmittel ein und erfasst insbesondere auch Gele und pastöse Zusammensetzungen. Insbesondere schließt der Begriff auch Nicht-Newtonsche Flüssigkeiten, die eine Fließgrenze besitzen, ein.

Alle im Zusammenhang mit den hierin beschriebenen Bestandteilen des Waschmittels angegeben Mengenangaben beziehen sich, sofern nichts anderes angegeben ist, auf Gew.% jeweils bezogen auf das Gesamtgewicht der Zusammensetzung. Des Weiteren beziehen sich derartige Mengenangaben, die sich auf mindestens einen Bestandteil beziehen, immer auf die Gesamtmenge dieser Art von Bestandteil, die im Waschmittel enthalten ist, sofern nicht explizit etwas anderes angegeben ist. Das heißt, dass sich derartige Mengenangaben, beispielsweise im Zusammenhang mit "mindestens einem nichtionischen Tensid", auf die Gesamtmenge von nichtionischen Tensiden die im Waschmittel enthalten ist, beziehen.

"Mindestens ein", wie hierin verwendet, bezieht sich auf 1 oder mehr, beispielsweise 1, 2, 3, 4, 5, 6, 7, 8, 9 oder mehr. Im Zusammenhang mit Bestandteilen der hierin beschriebenen Zusammensetzungen bezieht sich diese Angabe nicht auf die absolute Menge an Molekülen sondern auf die Art des Bestandteils. "Mindestens ein nichtionisches Tensid" bedeutet daher beispielsweise ein oder mehrere verschiedene nichtionische Tenside, d.h. eine oder mehrere verschiedene Arten von nichtionischen Tensiden. Zusammen mit Mengenangaben beziehen sich die Mengenangaben auf die Gesamtmenge der entsprechend bezeichneten Art von Bestandteil, wie bereits oben definiert.

"Im Wesentlichen frei" wie hierin verwendet, bedeutet, dass die jeweilige Verbindung in weniger als 0,01 Gew.-%, bevorzugt 0,001 Gew.-%, stärker bevorzugt 0,0001 Gew.-%, am stärksten bevorzugt gar nicht in der jeweiligen Komponente oder Zusammensetzung enthalten ist.

Sind Verbindungen in der vorliegenden Erfindung als substituiert beschrieben, so sind die möglichen Substituenten dem Fachmann bekannt. Insbesondere bevorzugt, falls nicht explizit anders angegeben, sind die Substituenten ausgewählt aus -F, -Cl, -Br, -I, -OH, =O, -OR', -NH₂, -NHR¹, -NR¹₂ und -COOR¹, wobei R¹ ein Alkylrest mit 1 bis 10 Kohlenstoffatomen ist.

Als "Ligand" werden in der Komplexchemie solche Moleküle bezeichnet, die in Gegenwart eines als Zentralatom oder Zentralion fungierenden Stoffes (z.B. Metallion) unter Ausbildung eines Komplexes an diesen Stoff gebunden sind. Als "freier Ligand" oder "unkomplexierter Ligand" liegt der Ligand in nichtkomplexierter Form ungebunden ohne Gruppierung um ein Zentralatom oder Zentralion vor.

Die Catechol-Metallkomplexverbindungen der Formel (I) können als Komplexkationen (q > r · p), Komplexanion (q < r · p) oder als neutraler Komplex (q = r · p) vorliegen. Im ersten Fall wird die kationische Ladung des Komplexkations durch ein entsprechendes Anion-Äquivalent unter Wahrung der Elektroneutralität kompensiert, das bevorzugt unter den bevorzugt geeigneten Anionen für X⁻ der Formel (I) ausgewählt wird (*vide infra*)*.*

Im zweiten Fall wird die anionische Ladung des Komplexkations durch ein entsprechendes Kation-Äquivalent unter Wahrung der Elektroneutralität kompensiert.

Die Reste R¹ und R² gemäß Formel (I) stehen unabhängig voneinander für einen besagten C₁-C₂₀-Kohlenwasserstoffrest. Die Kohlenwasserstoffreste mit 1 bis 20 Kohlenstoffatomen in Formel (I) können linear oder verzweigt, gesättigt oder ungesättigt, cyclisch oder alicyclisch oder aromatisch sein. Solche Reste sind bevorzugt ausgewählt aus C₁-C₂₀-Alkyl, C₁-C₂₀-Alkenyl, C₆-C₂₀-Aryl, Alkylaryl mit insgesamt 7 bis 20 Kohlenstoffatomen (z.B. Benzyl), der jeweils gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X⁻, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen.

Bevorzugt geeignete Catechol-Metallkomplexverbindungen der Formel (I) sind dadurch gekennzeichnet, dass in Formel (I) die Reste R¹ und R² unabhängig voneinander für eine Alkylgruppe, eine Alkoxyalkylgruppe, eine Hydroxyalkylgruppe, eine Hydroxyalkyloxyalkyl-Gruppe, (N-Hydroxyethyl)-aminoethyl, (N-Methoxyethyl)-aminoethyl oder (N-Ethoxyethyl)-aminoethyl, oder eine aromatische Gruppe stehen.

Bevorzugte Alkylgruppen sind gemäß Formel (I) lineare (C₁-C₁₀)-Alkylgruppen oder verzweigte (C₃-C₁₀)-Alkylgruppen oder C₅-C₆-Cycloalkyl. Methyl, Ethyl, n-Propyl, Isopropyl, n-Butyl, sec-Butyl, tert-Butyl, iso-Butyl, n-pentyl, iso-Pentyl, Neopentyl, Hexyl sind besonders bevorzugte Alkylgruppen gemäß Formel (I) (äußerst bevorzugt Methyl, Ethyl, n-Propyl, iso-Propyl).

Bevorzugte Alkenylgruppen sind gemäß Formel (I) Allyl, Vinyl, Butenyl.

Bevorzugte Alkoxyalkylgruppen der Formel (I) sind Methoxyethyl, Methoxypropyl, (2-Methoxy)-ethoxyethyl, Ethoxyethyl, Ethoxypropyl oder (2-Ethoxy)-ethoxyethyl.

Bevorzugte Hydroxyalkylgruppen sind gemäß Formel (I) 2-Hydroxyethyl, 3-Hydroxypropyl, 2-Hydroxypropyl, 1,2-Dihydroxypropyl.

Bevorzugte Hydroxyalkyloxyalkyl-Gruppen gemäß Formel (I) sind 2-Hydroxyethoxyethyl.

Besonders bevorzugt stehen die Reste R¹ und R² der Formel (I) unabhängig voneinander für Methyl, Ethyl, n-Propyl, Isopropyl, n-Butyl, sec-Butyl, tert-Butyl, iso-Butyl, n-pentyl, iso-Pentyl, Neopentyl, Hexyl, Allyl, Butenyl, 2-Hydroxyethyl, 2-Hydroxypropyl, 3-Hydroxypropyl, 1,2-Dihydroxypropyl, 2-Methoxyethyl, 2-Ethoxyethyl, 2-Methoxypropyl, 3-Methoxypropyl, 2-Ethoxypropyl, 3-Ethoxypropyl, 2-(N-Hydroxyethyl)-aminoethyl, 2-(N-Methoxyethyl)-aminoethyl oder 2-(N-Ethoxyethyl)-aminoethyl, Benzyl oder Phenyl.

Es hat sich als ganz besonders bevorzugt erwiesen, wenn in Formel (I) die Reste R¹ und R² gleich sind. Am bevorzugtesten stehen R¹ und R² für Methyl, Ethyl, n-Propyl, Isopropyl, n-Butyl, sec-Butyl, tert-Butyl, iso-Butyl, n-pentyl, iso-Pentyl, Neopentyl, Hexyl, 2-Hydroxyethyl, 2-Hydroxypropyl, 3-Hydroxypropyl, 2-Methoxyethyl, 2-Ethoxyethyl, 2-Methoxypropyl, 3-Methoxypropyl, 2-Ethoxypropyl, 3-Ethoxypropyl. Methyl, Ethyl, n-Propyl, iso-Propyl sind äußerst bevorzugte Gruppen für R¹ und R² der Catechol-Metallkomplexverbindungen der Formel (I).

X⁻ wird gemäß Formel (I) vorzugsweise aus der Gruppe umfassend Lactat, Citrat, Tartrat, Succinat, Perchlorat, Tetrafluoroborat, Hexafluorophosphat, Alkylsulfonat, Alkylsulfat, Hydrogensulfat, Sulfat, Dihydrogenphosphat, Hydrogenphosphat, Phosphat, Isocyanat, Rhodanid, Nitrat, Fluorid, Chlorid, Bromid, Hydrogencarbonat und Carbonat sowie Mischungen aus mindestens zweien von diesen ausgewählt, wobei der Ladungsausgleich bei Anwesenheit mehrwertiger Anionen durch die Anwesenheit entsprechend mehrerer kationischer Grundgerüste der allgemeinen Formel (I) oder gegebenenfalls durch die Anwesenheit zusätzlicher Kationen wie Natrium- oder Ammoniumionen gewährleistet werden kann.

Die gestrichelten Linien gemäß Formel (I) repräsentieren koordinative Bindungen des Liganden an das Metallkation M.

Höchst bevorzugte Catechol-Verbindungen der Formel (I) sind die Verbindungen der Formeln (I-a) und/oder (I-b) 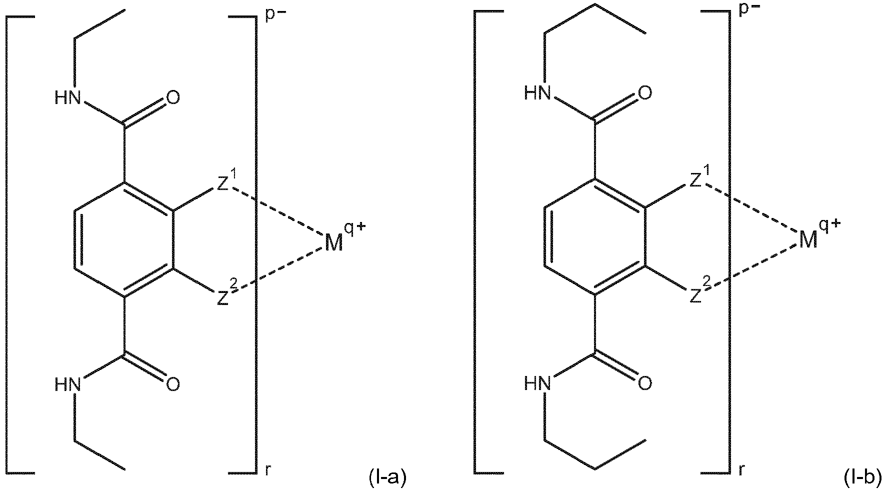 in denen Z¹, Z², p, r, q und M gemäß Formel (I) definiert sind.

Die Catechol-Metallkomplexverbindungen der vorliegenden Erfindung enthalten ein Metallion M, das für ein Metallkation eines Übergangsmetalls oder Lanthanoids steht. Unter einem Übergangsmetall versteht der Fachmann Metalle aus den Übergangselementen des Periodensystems der Elemente gemäß der nach IUPAC-Regel I-3.8.2 der Anorganischen Chemie gegebenen Definition. Lanthanoide sind dem Fachmann als Lanthan und die auf Lanthan folgenden Elemente der Seltenerdmetalle mit den Ordnungszahlen 58 bis 70 bekannt.

Bevorzugte Catechol-Metallkomplexverbindungen der Formeln (I), (I-a) und (I-b) enthalten ein Metallkation M ausgewählt aus Ti, Zr, Hf, V, Nb, Ta, Cr. Mo, W, Mn, Fe, Ru, Co, Ni, Cu, Zn, Ce, Sm oder Hydraten dieser Metallionen. Darunter sind wiederum solche Catechol-Metallkomplexverbindungen der Formel (I) besonders geeignet, bei denen in Formel (I) M als Metallkation ausgewählt ist aus einem Metallkation von Fe, Mn, Cr, Ni, Co, Ce, Cu oder Hydraten dieser Metallionen (insbesondere ausgewählt aus Fe(II), Fe(III), Mn(II), Mn(IV), Ni(II), Co(II), Co(III), Ce(III), Cu(II) oder Hydraten dieser Metallionen).

Die Catechol-Metallkomplexverbindungen der der Formeln (I), (I-a) und (I-b) sind farbig. Bevorzugte Catechol-Metallkomplexverbindungen der Formel (I) sind dadurch gekennzeichnet, dass sie Licht in einer Wellenlänge von 400 bis 800 nm absorbieren, gemessen mittels UV-VIS-Spektralphotometer (z.B. Specord^{®} S 600 mit Photodiodenarray der Firma Analytik Jena AG) bei einer Konzentration des Komplexes von 10⁻⁵ mol/L in Wasser bei 20°C, einem pH-Wert von 8 und einer Schichtdicke von 1 cm.

Die Catechol-Metallkomplexverbindungen der Formel (I) lassen sich bereitstellen, in dem 0,1·r mmol freier Ligand in Form der korrespondierenden Catecholverbindung in 10 mL Methanol gelöst werden. Dazu gibt man 0,1·r mmol KOH (als 0,5M Lösung in Methanol). 0,1 mmol des Metallchlorids (r ist wie in Formel (I) definiert (*vide supra*)) werden in 4 mL Methanol gelöst, diese Lösung wird zur ersten Lösung zugegeben. Man rührt für eine Stunde, engt die resultierende Lösung auf 4 mL ein und gibt zu ihr 50 mL Diethylether hinzu. Der ausgefällte Metallkomplex wird durch Filtration isoliert.

Ein erster Erfindungsgegenstand ist ein Waschmittel, insbesondere Flüssigwaschmittel, enthaltend jeweils bezogen auf das Gesamtgewicht des Waschmittels
- in einer Gesamtmenge von 0,001 bis 10,0 Gew.-%, bevorzugt 0,01 bis 3,0 Gew.-%, mindestens eine Catechol-Metallkomplexverbindung der Formel (I) 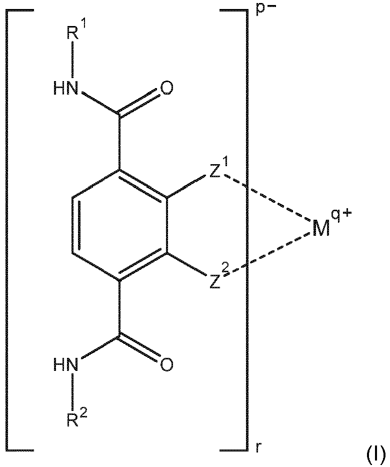 wobei
   - R¹ und R²: unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X⁻, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen,
   - Z¹ und Z²: unabhängig voneinander für OH oder O⁻ stehen,
   - M: steht für ein Metallkation eines Übergangsmetalls oder Lanthanoids, (insbesondere für ein Metallkation aus Ti, Zr, Hf, V, Nb, Ta, Cr. Mo, W, Mn, Fe, Ru, Co, Ni, Cu, Zn, Ce oder Sm),
   - q: als Ladungszahl des Metallkations M für eine Zahl 2, 3 oder 4 steht,
   - p: als Ladungszahl des Catecholliganden für eine Zahl 0, 1 oder 2 steht,
   - r: für eine Zahl 1, 2, 3 oder 4 steht,
   und
- mindestens ein Tensid, bevorzugt in einer Gesamtmenge von 2 bis 70 Gew.-%, insbesondere von 10 bis 65 Gew.-%, besonders bevorzugt von 15 bis 60 Gew.-%.

Das erfindungsgemäße Waschmittel ist bevorzugt ein Flüssigwaschmittel. Ein Stoff (z.B eine Zusammensetzung) ist gemäß Definition der Erfindung flüssig, wenn er bei 20°C und 1013 mbar im flüssigen Aggregatzustand vorliegt.

Es gelten für die Catechol-Komplexverbindungen der Formel (I) die im ersten Erfindungsgegenstand vorgenommenen Definitionen. Dabei sind die bevorzugten Ausführungsformen der Catechol-Metallkomplexverbindungen der Formel (I) des ersten Erfindungsgegenstandes ebenso bevorzugt in dem erfindungsgemäßen Waschmittel einsetzbar.

Die erfindungsgemäßen Waschmittel enthalten zwingend mindestens ein Tensid.

Das erfindungsgemäße Waschmittel enthält eine Gesamtmenge von 0,1 bis 70 Gew.-% mindestens eines Tensids. Es ist erfindungsgemäß bevorzugt, wenn das Waschmittel bezogen deren Gesamtgewicht eine Gesamtmenge von 5 bis 70 Gew.-%, bevorzugt von 10 bis 65 Gew.-%, besonders bevorzugt von 12 bis 60 Gew.-%, ganz besonders bevorzugt von 15 bis 60 Gew.-%, am bevorzugtesten von 20 bis 55 Gew.-%, mindestens eines Tensids enthält. Dabei eignen sich besonders ebenso bevorzugt folgende Tensidgesamtmengen des erfindungsmäßen Waschmittels: 10 bis 70 Gew.-% oder 12 bis 70 Gew.-% oder 15 bis 70 Gew.-% oder 20 bis 70 Gew.-% oder 5 bis 65 Gew.-% oder 10 bis 65 Gew.-% oder 12 bis 65 Gew.-% oder 15 bis 65 Gew.-% oder 20 bis 65 Gew.-% oder 5 bis 60 Gew.-% oder 10 bis 60 Gew.-% oder 12 bis 60 Gew.-% oder 15 bis 60 Gew.-% oder 20 bis 60 Gew.-% oder 5 bis 55 Gew.-% oder 10 bis 55 Gew.-% oder 12 bis 55 Gew.-% oder 15 bis 55 Gew.-% oder 20 bis 55 Gew.-%.

Das erfindungsgemäße Waschmittel enthält bevorzugt mindestens ein anionisches Tensid. Es ist bevorzugt, dass das anionische Tensid ausgewählt ist aus der Gruppe bestehend aus C₈-₁₈-Alkylbenzolsulfonaten, C₈₋₁₈-Olefinsulfonaten, C₁₂₋₁₈-Alkansulfonaten, C₈₋₁₈-Estersulfonaten, C₈₋₁₈-Alkylsulfaten, C₈₋₁₈-Alkenylsulfaten, Fettalkoholethersulfaten und Mischungen daraus. Besonders bevorzugt wird das anionische Tensid aus mindestens einem C₈₋₁₈-Alkylbenzolsulfonat ausgewählt.

Es hat sich gezeigt, dass sich diese Sulfonat- und Sulfat-Tenside besonders gut zur Herstellung stabiler Waschmittel eignen.

Es ist erfindungsgemäß bevorzugt, wenn das Waschmittel mindestens ein anionisches Tensid der Formel (T1) enthält 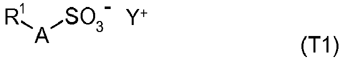 worin
- R¹: für einen linearen oder verzweigten, substituierten oder unsubstituierten Rest, ausgewählt aus C₈₋₁₈-Alkyl-, Aryl- oder C₈₋₁₈-Alkylarylresten und die Gruppierung -A- für eine chemische Bindung oder einen Rest -(OZ)ₙ-O- steht, worin OZ für eine Ethylenoxid- (EO) oder Propylenoxid- (PO) Gruppierung und n für eine ganze Zahl von 1 bis 50, vorzugsweise von 1 bis 20 und insbesondere von 2 bis 10, ganz besonders bevorzugt 2, 3, 4, 5, 6, 7 oder 8,
- Y⁺: für ein einwertiges Kation oder den n-ten Teil eines n-wertigen Kations, steht.

Dabei hat es sich erfindungsgemäß als geeignet herausgestellt, wenn besagtes Waschmittel mindestens ein solches Tensid der obigen Formel (T1) enthält, bei dem A gemäß Formel (T1) für die Struktureinheit -(OZ)ₙ-O- steht,
wobei OZ für eine Ethylenoxid- (EO) oder Propylenoxid- (PO) Gruppierung und n für eine ganze Zahl von 1 bis 50, vorzugsweise von 1 bis 20 und insbesondere von 2 bis 10, ganz besonders bevorzugt 2, 3, 4, 5, 6, 7 oder 8, steht,
und R¹ gemäß Formel (T1) für einen linearen oder verzweigten, substituierten oder unsubstituierten Alkylrest steht. Es ergeben sich daraus Alkylethersulfate mit der Formel (T1-1)

R¹-(OZ)ₙ-O-SO₃- Y⁺ (T1-1).

In dieser Formel (T1-1) steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₁₃-Oxoalkoholen. Y⁺ ist wie zuvor in Formel (T1) definiert.

OZ steht gemäß Formel (T1-1) für eine Ethylenoxid- (EO) oder Propylenoxid- (PO) Gruppierung, vorzugsweise für eine Ethylenoxidgruppierung. Der Index n steht gemäß Formel (I-1) für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. Y⁺ steht gemäß Formel (T1-1) für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen Y⁺ können ausgewählt sein aus NH₄⁺, ½ Zn²⁺,½ Mg²⁺,½ Ca²⁺,½ Mn²⁺, und deren Mischungen.

Waschmittel können als Verbindung der Formel (T1) bzw. als Verbindung der Formel (T1-1) mindestens ein Alkylethersulfat ausgewählt aus Fettalkoholethersulfaten der Formel (T1-2) enthalten 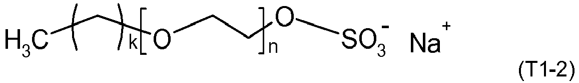 mit k = 11 bis 19, n = 2, 3, 4, 5, 6, 7 oder 8. Ganz besonders bevorzugte Vertreter sind Na-C₁₂-₁₄ Fettalkoholethersulfate mit 2 EO (k = 11-13, n = 2 in Formel A-1). Der angegebenen Ethoxylierungsgrad stellt einen statistischen Mittelwert dar, der für ein spezielles Produkt eine ganze oder eine gebrochene Zahl sein kann. Die angegebenen Alkoxylierungsgrade stellen statistische Mittelwerte dar, die für ein spezielles Produkt eine ganze oder eine gebrochene Zahl sein können. Bevorzugte Alkoxylate/Ethoxylate weisen eine eingeengte Homologenverteilung auf (narrow range ethoxylates, NRE).

Es ist erfindungsgemäß besonders bevorzugt, wenn die erfindungsgemäßen Waschmittel als anionisches Tensid der Formel (T1) mindestens eine Verbindung der Formel (T1-3) enthalten 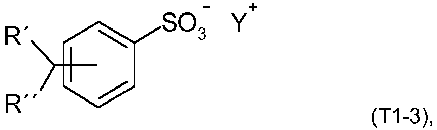 in der
R' und R" unabhängig H oder Alkyl sind und zusammen 9 bis 19, vorzugsweise 9 bis 15 und insbesondere 9 bis 13 C-Atome enthalten, und Y⁺ ein einwertiges Kation oder den n-ten Teil eines n-wertigen Kations (insbesondere Na⁺) bedeuten (ausgehend von Formel (T1): -A- = chemische Bindung, R¹ = lineares oder verzweigtes Alkylaryl, Y⁺ = Na⁺). Ein ganz besonders bevorzugter Vertreter lässt sich durch die Formel (T1-3a) beschreiben:

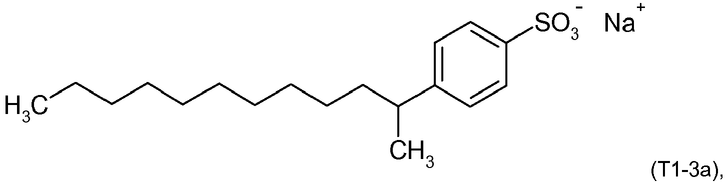

Die erfindungsgemäßen Waschmittel enthalten bevorzugt als Tensid der Formel (T1) eine Kombination aus
- mindestens einem Fettalkoholethersulfat der Formel (T1-1)

   R¹-(OZ)ₙ-O-SO₃- Y*⁺ (T1-1).

   worin
   R¹ steht für einen linearen oder verzweigten (C₈-C₁₈)-Alkylrest,
   OZ steht für eine Ethylenoxid- (EO) oder eine Propylenoxid- (PO) Gruppierung,
   n steht für eine ganze Zahl von 1 bis 50, vorzugsweise von 1 bis 20 und insbesondere von 2 bis 10, und
   Y* ⁺ steht für ein einwertiges Kation oder den n-ten Teil eines n-wertigen Kations,
   und
- mindestens einem linearen oder verzweigten Alkylbenzolsulfonat der Formel A-4 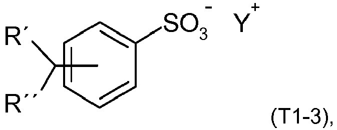 in der R' und R" zusammen 9 bis 19, vorzugsweise 11 bis 15 und insbesondere 11 bis 13 C-Atome enthalten und Y⁺ für ein einwertiges Kation oder den n-ten Teil eines n-wertigen Kations (insbesondere für Na⁺) steht,
enthalten.

Das flüssige Waschmittel kann als anionisches Tensid auch Seifen enthalten. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Wenn das erfindungsgemäße Waschmittel anionisches Tensid enthält, ist es wiederum bevorzugt, dass bezogen auf das Gesamtgewicht des Waschmittels anionisches Tensid in einer Gesamtmenge von 4 bis 70 Gew.-%, insbesondere 10 bis 50 Gew.-%, weiter bevorzugt von 15 bis 40 Gew.-%, enthalten ist.

Das erfindungsgemäße Waschmittel kann als Tensid bevorzugt mindestens ein nichtionisches Tensid enthalten. Bevorzugte Waschmittel enthalten somit mindestens ein anionisches Tensid und/oder mindestens ein nichtionisches Tensid enthalten ist. In einer besonders bevorzugten Ausführungsform enthält das erfindungsgemäße Waschmittel neben mindestens einem anionischen Tensid zusätzlich mindestens ein nichtionisches Tensid. Geeignete zusätzliche nichtionische Tenside umfassen alkoxylierte Fettsäurealkylester, alkoxylierte Fettsäureamide, hydroxylierte Alkylglykolether, Polyhydroxyfettsäureamide, Alkylphenolpolyglycolether, Aminoxide, Alkyl(poly)glucoside und Mischungen daraus.

Besonders bevorzugt enthält das erfindungsgemäße Mittel als nichtionisches Tensid mindestens eine Verbindung der Formel (T2)

R²-O-(XO)ₘ-H, (T2)

in der
- R²: für einen linearen oder verzweigten C₈-C₁₈-Alkylrest, einen Arylrest oder Alkylarylrest,
- XO: unabhängig voneinander für eine Ethylenoxid- (EO) oder Propylenoxid- (PO) Gruppierung,
- m: für ganze Zahlen von 1 bis 50 stehen.

Besonders bevorzugte Reste R² der Formel (T2) sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₈-C₁₈-Oxoalkoholen.

XO steht gemäß Formel (T2) vorzugsweise für eine Ethylenoxidgruppierung.

Der Index m steht gemäß Formel (T2) vorzugsweise für eine Zahl von 1 bis 20 und insbesondere von 2 bis 10. Ganz besonders bevorzugt steht m für die Zahlen 2, 3, 4, 5, 6, 7 oder 8.

Als nichtionisches Tensid werden vorzugsweise alkoxylierte, vorteilhafterweise ethoxylierte, insbesondere primäre Alkohole mit vorzugsweise 8 bis 18 C-Atomen und durchschnittlich 4 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 4 bis 8 EO pro Mol Alkohol bevorzugt. Zu den bevorzugten ethoxylierten Alkoholen gehören beispielsweise C₁₂₋₁₄-Alkohole mit 4 EO oder 7 EO, C₉₋₁₁-Alkohol mit 7 EO, C₁₃₋₁₅-Alkohole mit 5 EO, 7 EO oder 8 EO, C₁₂₋₁₈-Alkohole mit 5 EO oder 7 EO und Mischungen aus diesen. Bevorzugte Alkoholethoxylate weisen eine eingeengte Homologenverteilung auf (narrow range ethoxylates, NRE). Zusätzlich zu diesen oder anstelle dieser bevorzugten nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Insbesondere bevorzugt enthält das erfindungsgemäße Waschmittel einen C₁₂₋₁₈-Fettalkohol mit 7 EO oder einen C₁₃₋₁₅-Oxoalkohol mit 7 EO als nichtionisches Tensid.

Als Aminoxid sind prinzipiell alle im Stand der Technik für diese Zwecke etablierten Aminoxide also Verbindungen, die die Formel R¹R²R³NO aufweisen, worin jedes R¹, R² und R³ unabhängig von den anderen eine gegebenenfalls substituierte C₁-C₃₀ Kohlenwasserstoffkette ist, einsetzbar. Besonders bevorzugt eingesetzte Aminoxide sind solche in denen R¹ C₁₂-C₁₈ Alkyl und R² und R³ jeweils unabhängig C₁-C₄ Alkyl sind, insbesondere C₁₂-C₁₈ Alkyldimethylaminoxide. Beispielhafte Vertreter geeigneter Aminoxide sind N-Kokosalkyl-N,N-dimethylaminoxid, N-Talgalkyl-N,N-dihydroxyethylaminoxid, Myristyl-/Cetyldimethylaminoxid oder Lauryldimethylaminoxid.

Es ist erfindungsgemäß bevorzugt, wenn die erfindungsgemäßen Waschmittel bezogen auf deren Gesamtgewicht nichtionisches Tensid in einer Gesamtmenge zwischen 0 und 35 Gew.-% und vorzugsweise von 5 bis 30 Gew.-%, ganz besonders bevorzugt von 7 bis 28 Gew.-%, jeweils bezogen auf das gesamte Mittel, enthalten.

Bevorzugte Waschmittel der vorliegenden Erfindung weisen bei 20°C einen pH-Wert von 6 bis 11,5, bevorzugt 6,5 bis 9,5, stärker bevorzugt 7,0 bis 9,0 , auf.

Die erfindungsgemäßen Waschmittel weisen insbesondere eine hervorragende Reinigungswirkung an bleichbaren Verschmutzungen auf, wenn sie zusätzlich mindestens eine Verbindung gemäß Formel (II) enthalten, welche nicht an ein Metallkation M eines Übergangsmetalls oder Lanthanoids gemäß Formel (I) (insbesondere nicht an ein Metallkation der unter Formel (I) bevorzugt genannten Vertreter von M) komplexiert ist. Bevorzugte Waschmittel sind daher dadurch gekennzeichnet, dass zusätzlich mindestens eine freie Catecholverbindung der Formel (II) oder deren Salz enthalten sind 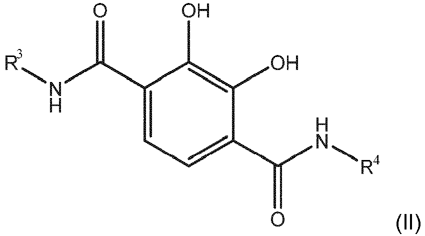 wobei
R³ und R⁴ unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X⁻, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen,
mit der Maßgabe, dass die Catecholverbindung der Formel (II) und deren Salz von Verbindungen der Formel (I) (*vide supra*) verschieden sind.

Die "freie Catecholverbindung" der Formel (II) liegt im Rahmen der obigen Maßgabe als unkomplexierter Ligand vor und/oder als Komplex, der von Verbindungen der Formel (I) verschieden ist. Das Salz der freien Catecholverbindung der Formel (II) liegt an mindestens einer der direkt an den Catechol-Phenylring bindenden OH-Gruppen deprotoniert vor, wobei die entstehende negative Ladung durch ein entsprechendes Kation-Äquivalent neutralisiert wird. Dabei kann das Salz der freien Catecholverbindung in dem erfindungsgemäßen Waschmittel dissoziiert vorliegen und/oder ein Komplex an ein Kation-Äquivalent als Zentralion sein, wobei besagtes Kation-Äquivalent von dem Metallkation M der Formel (I) verschieden ist.

Bevorzugte Kation-Äquivalente des Salzes der freien Catecholverbindung der Formel (II) werden ausgewählt aus Alkalimetallion (bevorzugt Na⁺ oder K⁺), Erdalkalimetallion (bevorzugt Ca²⁺ oder Mg²⁺), Alkanolammoniumion. Als Alkanolammoniumionen kommen bevorzugt 2-Ammoninoethan-1-ol, Tris(2-hydroxyethyl)ammonium, 3-Ammoniopropan-1-ol, 4-Ammoninbutan-1-ol, 5-Amnoniopentan-1-ol, 1-Ammoniopropan-2-ol, 1-Ammoniobutan-2-ol, 1-Ammoniopentan-2-ol, 1-Ammoniopentan-3-ol, 1-Ammoniopentan-4-ol, 3-Ammonio-2-methylpropan-1-ol, 1-Ammonio-2-methylpropan-2-ol, 3-Ammoniopropan-1,2-diol, 2-Ammonio-2-methylpropan-1,3-diol (insbesondere 2-Ammonioethan-1-ol, Tris(2-hydroxyethyl)ammonium, 2-Ammonio-2-methylpropan-1-ol, 2-Ammonio-2-methyl-propan-1,3-diol), oder Mischungen davon in Betracht.

Bevorzugt wird in dem erfindungsgemäßen Waschmitteln, dass in Formel (II) die Reste R³ und R⁴ unabhängig voneinander für eine Alkylgruppe, eine Alkoxyalkylgruppe, eine Hydroxyalkylgruppe, eine Hydroxyalkyloxyalkyl-Gruppe, (N-Hydroxyethyl)-aminoethyl, (N-Methoxyethyl)-aminoethyl oder (N-Ethoxyethyl)-aminoethyl, oder eine aromatische Gruppe stehen (bevorzugt für Methyl, ethyl, n-Propyl, Isopropyl, n-Butyl, sec-Butyl, tert-Butyl, iso-Butyl, n-pentyl, iso-Pentyl, Neopentyl, Hexyl, 2-Hydroxyethyl, 2-Hydroxypropyl, 3-Hydroxypropyl, (N-Hydroxyethyl)-aminoethyl, (N-Methoxyethyl)-aminoethyl oder (N-Ethoxyethyl)-aminoethyl oder Phenyl).

Bevorzugte Verbindungen der Formel (II) sind solche, bei denen die Reste R³ und R⁴ gleich sind.

Die bevorzugtem Reste R³ und R⁴ der Formel (II) werden unter den bevorzugten Resten R¹ und R² der Formel (I) ausgewählt.

Ganz besonders bevorzugt werden die Reste R³ und R⁴ der Formel (II) abhängig von den Resten R¹ und R² der Formel (I) ausgewählt, wobei am bevorzugtesten die Reste R¹, R², R³ und R⁴ identisch sind.

Es ist besonders bevorzugt, wenn das erfindungsgemäße Waschmittel die freie Catecholverbindung der Formel (II) in einer Gesamtmenge von 0,01 bis 10,0 Gew.-%, bevorzugt von 0,1 bis 5,0 Gew.-%, enthält.

Das erfindungsgemäße Waschmittel enthält als weiteren Bestandteil bevorzugt Wasser. Besonders bevorzugte Waschmittel enthalten - bezogen auf ihr Gewicht - Wasser in einer Gesamtmenge von 5 bis 90 Gew.-%, bevorzugt 10 bis 85 Gew.-%, besonders bevorzugt 25 bis 75 Gew.-% und insbesondere 35 bis 65 Gew.-%. Alternativ kann es sich bei den Waschmitteln um wasserarme Waschmittel handeln, wobei die Gesamtmenge an Wasser in einer bevorzugten Ausführungsform zwischen 0 und 25 Gew.-%, insbesondere zwischen 1 und 20 Gew.-% und besonders bevorzugt zwischen 2 und 18 Gew.-%, jeweils bezogen auf das gesamte Waschmittel, beträgt.

Der Wassergehalt wie hierin definiert bezieht sich auf den mittels der Karl Fischer Titration ermittelten Wassergehalt (Angewandte Chemie 1935, 48, 394-396; ISBN 3-540-12846-8 Eugen Scholz).

Das erfindungsgemäße Waschmittel ist bevorzugt transparent oder transluzent. Weist ein erfindungsgemäßes Waschmittel im spektralen Bereich zwischen 380 nm und 780 nm eine auf die Referenzmessung bezogene rest-Lichtleistung (Transmission) von mindestens 20 % auf, gilt sie als transparent im Sinne der Erfindung.

Die Transparenz des erfindungsgemäßen Waschmittels kann mit verschiedenen Methoden ermittelt werden. Die Nephelometric Turbidity Unit (Nephelometrischer Trübungswert; NTU) wird häufig als Messwert für Transparenz herangezogen. Sie ist eine z.B. in der Wasseraufbereitung verwendete Einheit für Trübungsmessungen z.B. in Flüssigkeiten. Sie ist die Einheit einer mit einem kalibrierten Nephelometer gemessenen Trübung. Hohe NTU-Werte werden für getrübte Waschmittel gemessen, wogegen niedrige Werte für klare, transparente Waschmittel bestimmt werden.

Der Einsatz des Turbidimeters vom Typ HACH Turbidimeter 2100Q der Fa. Hach Company, Loveland, Colorado (USA) erfolgt dabei unter Verwendung der Kalibriersusbstanzen StablCal Solution HACH (20 NTU), StablCal Solution HACH (100 NTU) und StablCal Solution HACH (800 NTU), alle können ebenfalls von der Firma Hach Company bestellt werden. Die Messung wird in einer 10 ml Messküvette mit Kappe mit der zu untersuchenden Zusammensetzung befüllt und die Messung bei 20 °C durchgeführt.

Bei einem NTU-Wert (bei 20°C) von 60 oder mehr weisen Waschmittel mit dem bloßen Auge erkennbar im Sinne der Erfindung eine wahrnehmbare Trübung auf. Daher ist es bevorzugt, wenn die erfindungsgemäßen Waschmittel einen NTU-Wert (bei 20°C) von höchstens 120, bevorzugter höchstens 110, bevorzugter höchstens 100, besonders bevorzugt von höchstens 80, aufweisen.

Im Rahmen der vorliegenden Erfindung wurde die Transparenz der erfindungsgemäßen Waschmittel durch eine Transmissionsmessung im visuellen Lichtspektrum über einen Wellenlängenbereich von 380 nm bis 780 nm bei 20°C bestimmt. Dazu wird zunächst eine Referenzprobe (Wasser, vollentsalzt) in einem Photometer (Fa. Specord S 600 von AnalytikJena) mit einer im zu untersuchendem Spektrum transparenten Küvette (Schichtdicke 10 mm) vermessen. Anschließend wird die Küvette mit einer Probe des erfindungsgemäßen Waschmittels befüllt und abermals vermessen. Dabei wird die Probe in flüssigem Zustand eingefüllt und falls nötig in der Küvette verfestigt und dann vermessen.

Es ist bevorzugt, wenn das erfindungsgemäße transparente Waschmittel eine Transmission (20°C) von bevorzugter mindestens 25 %, bevorzugter mindestens 30%, bevorzugter mindestens 40 %, insbesondere von mindestens 50 %, besonders bevorzugt von mindestens 60 %, aufweist.

Es ist ganz besonders bevorzugt, wenn das erfindungsgemäße transparente Waschmittel eine Transmission (bei 20°C) von mindestens 30 % (insbesondere von mindestens 40 % bevorzugter von mindestens 50 %, besonders bevorzugt von mindestens 60 %) und einen NTU-Wert (bei 20°C) von höchstens 120 (bevorzugter höchstens 110, bevorzugter höchstens 100, besonders bevorzugt von höchstens 80) aufweist.

Im Rahmen einer erfindungsgemäß bevorzugten Ausführungsform ist das erfindungsgemä0e Waschmittel flüssig, weist eine Fließgrenze auf und enthält das erfindungsgemäße Waschmittel zusätzlich suspendierte Feststoffpartikel (im Folgenden auch als Partikel bezeichnet). Als solche suspendierten Feststoffpartikel sind feste Stoffe zu verstehen, die sich bei 20°C nicht in der kontinuierlichen Phase des erfindungsgemäßen Waschmittels lösen und als separate Phase vorliegen.

Die Partikel werden bevorzugt ausgewählt aus Polymeren, Perlglanzpigmenten, Mikrokapseln, Speckles oder Mischungen daraus.

Mikrokapseln im Sinne der vorliegenden Erfindung umfassen jede dem Fachmann bekannte Art von Kapsel, insbesondere jedoch Kern-Schale-Kapseln und Matrixkapseln. Matrixkapseln sind poröse Formkörper, die eine Struktur ähnlich zu einem Schwamm aufweisen. Kern-Schale-Kapseln sind Formkörper, die einen Kern und eine Schale aufweisen. Als Mikrokapseln sind solche Kapseln geeignet, die einen mittleren Durchmesser X_{50,3} (Volumenmittel) von 0.1 bis 200 µm, bevorzugt von 1 bis 100 µm, weiter bevorzugt 5 bis 80 µm, besonders bevorzugt von 10 bis 50 µm und insbesondere von 15 bis 40 µm aufweisen. Der mittlere Teilchengrößendurchmesser X _{50,3} wird durch Siebung oder mittels eines Partikelgrößenanalysators Camsizer der Fa. Retsch bestimmt.

Die Mikrokapseln der Erfindung enthalten bevorzugt mindestens einen Wirkstoff, bevorzugt mindestens einen Riechstoff. Diese bevorzugten Mikrokapseln sind Parfummikrokapseln.

In einer bevorzugten Ausführungsform der Erfindung weisen die Mikrokapseln eine semipermeable Kapselwand (Schale) auf.

Eine semipermeable Kapselwand im Sinne der vorliegenden Erfindung ist eine Kapselwand, die halbdurchlässig ist, also kontinuierlich über die Zeit kleine Mengen des Kapselkerns freisetzt, ohne dass die Kapsel z.B. durch Reibung beschädigt bzw. geöffnet wurde. Solche Kapseln, setzen über einen längeren Zeitraum kontinuierlich kleine Mengen des in der Kapsel befindlichen Wirkstoffs, z.B. Parfum, frei.

In einer weiteren bevorzugten Ausführungsform der Erfindung weisen die Mikrokapseln eine impermeable Schale auf. Eine impermeable Schale im Sinne der vorliegenden Erfindung ist eine Kapselwand, die im Wesentlichen undurchlässig ist, also den Kapselkern erst durch Beschädigung bzw. Öffnung der Kapsel freigibt. Solche Kapseln enthalten im Kapselkern signifikante Mengen des mindestens einen Riechstoffs, so dass bei Beschädigung bzw. bei Öffnung der Kapsel ein sehr intensiver Duft bereitgestellt wird. Die so erzielten Duftintensitäten sind in der Regel so hoch, dass geringere Mengen der Mikrokapseln eingesetzt werden können um die gleiche Duftintensität zu erzielen wie bei herkömmlichen Mikrokapseln.

In einer bevorzugten Ausführungsform der Erfindung enthält das erfindungsgemäße Waschmittel sowohl Mikrokapseln mit semipermeabler Schale, also auch Mikrokapseln mit impermeabler Schale. Durch den Einsatz von beiden Kapsel-Typen kann über den gesamten Wäschezyklus eine deutlich verbesserte Duftintensität bereitgestellt werden.

In einer weiteren bevorzugten Ausführungsform der Erfindung kann das erfindungsgemäße Waschmittel auch zwei oder mehr verschiedene Mikrokapseltypen mit semipermeabler oder impermeabler Schale enthalten.

Als Materialen für die Schale der Mikrokapseln kommen üblicherweise hochmolekulare Verbindungen in Frage wie zum Beispiel Eiweißverbindungen, wie zum Beispiel Gelatine, Albumin, Casein und andere, Cellulose-Derivate, wie zum Beispiel Methylcellulose, Ethylcellulose, Celluloseacetat, Cellulosenitrat, Carboxymethylcellulose und andere sowie vor allem auch synthetische Polymere wie zum Beispiel Polyamide, Polyethylenglycole, Polyurethane, Epoxydharze und andere. Bevorzugt dient als Wandmaterial, also als Schale, Melamin-Formaldehyd-Polymer, Melamin-Harnstoff-Polymer, Melamin-Harnstoff-Formaldehyd-Polymer, Polyacrylat-Polymer oder Polyacrylat-Copolymer. Erfindungsgemäße Kapseln werden beispielsweise, aber nicht ausschließlich, in US 2003/0125222 A1, DE 10 2008 051 799 A1 oder WO 01/49817 beschrieben.

Bevorzugte Melamin-Formaldehyd-Mikrokapseln werden hergestellt, in dem man Melamin-Formaldehyd-Vorkondensate und/oder deren C₁-C₄-Alkylether in Wasser, in dem die mindestens eine Geruchsmodulatorverbindung und ggf. weitere Inhaltsstoffe, wie z.B. mindestens ein Riechstoff, in Gegenwart eines Schutzkolloids kondensiert. Geeignete Schutzkolloide sind z.B. Cellulosederivate, wie Hydroxyethylcellulose, Carboxymethylcellulose und Methylcellulose, Polyvinylpyrrolidon, Copolymere des N-Vinylpyrrolidons, Polyvinylalkohole, partiell hydrolysierte Polyvinylacetate, Gelatine, Gummi arabicum, Xanthangummi, Alginate, Pectine, abgebaute Stärken, Kasein, Polyacrylsäure, Polymethacrylsäure, Copolymerisate aus Acrylsäure und Methacrylsäure, sulfonsäuregruppenhaltige wasserlösliche Polymere mit einem Gehalt an Sulfoethylacrylat, Sulfoethylmethacrylat oder Sulfopropylmethacrylat, sowie Polymerisate von N-(Sulfoethyl)-maleinimid, 2-Acrylamido-2-alkylsulfonsäuren, Styrolsulfonsäuren und Formaldehyd sowie Kondensate aus Phenolsulfonsäuren und Formaldehyd.

Es ist bevorzugt, die erfindungsgemäß verwendeten Mikrokapseln an deren Oberfläche ganz oder teilweise mit mindestens einem kationischen Polymer zu beschichten. Entsprechend eignet sich als kationisches Polymer zur Beschichtung der Mikrokapseln mindestens ein kationisches Polymer aus Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-56, Polyquaternium-57, Polyquaternium-61, Polyquaternium-69, Polyquaternium-86. Ganz besonders bevorzugt ist Polyquaternium-7. Die im Rahmen dieser Anmeldung genutzte Polyquaternium-Nomenklatur der kationischen Polymere ist der Deklaration kationischer Polymere gemäß International Nomenclature of Cosmetic Ingredients (INCI-Deklaration) kosmetischer Rohstoffe entnommen.

Bevorzugt einsetzbare Mikrokapseln weisen mittlere Durchmesser X_{50,3} im Bereich von 1 bis 100 µm auf, vorzugsweise von 5 bis 95 µm, insbesondere von 10 bis 90 µm, zum Beispiel von 10 bis 80 µm.

Die den Kern beziehungsweise (gefüllten) Hohlraum umschließende Schale der Mikrokapseln hat bevorzugt eine durchschnittliche Dicke im Bereich von rund 5 bis 500 nm, vorzugsweise von rund 50 nm bis 200 nm, insbesondere von rund 70 nm bis etwa 180 nm.

Perlglanzpigmente sind Pigmente, die einen perlmuttartigen Glanz besitzen. Perlglanzpigmente bestehen aus dünnen Blättchen, die einen hohen Brechungsindex aufweisen und teilweise das Licht reflektieren sowie teilweise für das Licht transparent sind. Der perlmuttartige Glanz wird durch Interferenz des auf das Pigment treffenden Lichts erzeugt (Interferenzpigment). Perlglanzpigmente sind meist dünne Blättchen des oben genannten Materials, oder enthalten das o.g. Material als dünne mehrschichtige Filme oder als parallel angeordnete Bestandteile in einem geeigneten Trägermaterial.

Die erfindungsgemäß verwendbaren Perlglanzpigmente sind entweder natürliche Perlglanzpigmente wie z.B. Fischsilber (Guanin/Hypoxanthin-Mischkristalle aus Fischschuppen) oder Perlmutt (aus vermahlenen Muschelschalen), monokristalline blättchenförmige Perlglanzpigmente wie z.B. Bismutoxychlorid sowie Perglanzpigmente auf Basis von Glimmer sowie Glimmer/Metalloxid. Die letztgenannten Perlglanzpigmente sind Glimmer, die mit einem Metalloxidcoating versehen wurden.

Durch den Einsatz der Perlglanzpigmente in der erfindungsgemäßen Suspension werden Glanz und gegebenenfalls zusätzlich Farbeffekte erzielt.

Perlglanzpigmente auf Glimmer-Basis und auf Glimmer/Metalloxid-Basis sind erfindungsgemäß bevorzugt. Glimmer gehören zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiOz, Cr₂O₃ und Fe₂O₃. Durch entsprechende Beschichtung werden Interferenzpigmente sowie Farbglanzpigmente als erfindungsgemäße Perlglanzpigmente erhalten. Diese Perlglanzpigmentarten weisen neben einem glitzernden optischen Effekt zusätzlich Farbeffekte auf. Desweiteren können die erfindungsgemäß verwendbaren Perlglanzpigmente weiterhin ein Farbpigment enthalten, welches sich nicht von einem Metalloxid ableitet.

Die Korngröße der bevorzugt verwendeten Perlglanzpigmente liegt bevorzugt bei einem mittleren Durchmesser X_{50,3} (Volumenmittel) zwischen 1.0 und 100 µm, besonders bevorzugt zwischen 10.0 und 60.0 µm.

Unter Speckles sind im Sinne der Erfindung Makropartikel, insbesondere Makrokapseln, zu verstehen, die einen mittleren Durchmesser X_{50,3} (Volumenmittel) von mehr als 300 µm, insbesondere von 300 bis 1500 µm, bevorzugt von 400 bis 1000 µm, aufweisen.

Bei Speckles handelt es sich bevorzugt um Matrixkapseln. Die Matrix ist bevorzugt gefärbt. Die Matrixbildung erfolgt beispielsweise über Gelierung, Polyanion-Polykation-Wechselwirkungen oder Polyelektrolyt-Metallion-Wechselwirkungen und ist im Stand der Technik genauso wie die Herstellung von Partikeln mit diesen matrix-bildenden Materialien wohl bekannt. Ein beispielhaftes matrix-bildendes Material ist Alginat. Zur Herstellung Alginat-basierter Speckles wird eine wässrige Alginat-Lösung, welche ggf zusätzlich den einzuschließenden Wirkstoff bzw. die einzuschließenden Wirkstoffe enthält, vertropft und anschließend in einem Ca²⁺-Ionen oder Al³⁺-Ionen enthaltendem Fällbad ausgehärtet. Alternativ können anstelle von Alginat andere, matrixbildende Materialien eingesetzt werden.

Die erfindungsgemäßen Waschmittel enthalten bevorzugt zusätzlich mindestens einen weiteren Aktivstoff. Aktivstoffe im Sinne der vorliegenden Erfindung sind insbesondere:
- Textilpflegemittel wie Weichmacher, Phobier- und Imprägniermittel gegen Wasser und Wiederanschmutzungen, Bleichaktivatoren, Enzyme, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Antistatika, Bügelhilfsmittel, Quell- und Schiebefestmittel, UV-Absorber, kationische Polymere,
- Hautpflegemittel oder
- Parfüm(öl) oder Riechstoffe.

Das erfindungsgemäße Waschmittel bevorzugt im Wesentlichen frei von zusätzlichen Farbstoffen.

Bevorzugt ist mindestens ein Aktivstoff ausgewählt aus Enzymen, optischen Aufhellern, Gerüststoffen, Lösemitteln, Antiredepositionsmitteln, Farbtransferinhibitoren, Konservierungsmitteln, Parfüm oder Mischungen aus mindestens zwei der vorgenannten Aktivstoffe.

Das Waschmittel kann ferner ein von der Catecholverbindung gemäß Formel (II) verschiedenes zusätzliches Bleichmittel enthalten. In einer bevorzugten Ausführungsform ist im Wesentlichen kein weiteres Bleichmittel enthalten.

Es ist bevorzugt, wenn das erfindungsgemäße Waschmittel zusätzlich mindestens ein Enzym, insbesondere ausgewählt aus Protease, Amylase, Lipase, Mannanase, Cellulase, Pectatlyase oder Mischungen daraus, enthält.

"Variante" ist auf der Ebene der Proteine der zu "Mutante" entsprechende Begriff auf der Ebene der Nukleinsäuren. Bei den Vorgänger- oder Ausgangsmolekülen kann es sich um Wildtypenzyme handeln, das heißt solche, die aus natürlichen Quellen erhältlich sind. Es kann sich auch um Enzyme handeln, die an sich bereits Varianten darstellen, das heißt gegenüber den Wildtypmolekülen bereits verändert worden sind. Darunter sind beispielsweise Punktmutanten, solche mit Änderungen der Aminosäuresequenz, über mehrere Positionen oder längere zusammenhängende Bereiche, oder auch Hybridmoleküle zu verstehen, die aus einander ergänzenden Abschnitten verschiedener Wildtyp Enzyme zusammengesetzt sind.

Unter Aminosäureaustauschen sind Substitutionen einer Aminosäure gegen eine andere Aminosäure zu verstehen. Erfindungsgemäß werden solche Substitutionen unter Bezeichnung der Positionen, in der der Austausch erfolgt, gegebenenfalls kombiniert mit den betreffenden Aminosäuren im international gebräuchlichen Einbuchstabencode angegeben. "Austausch in Position 320" bedeutet beispielsweise, dass eine Variante in der Position, die in der Sequenz eines Referenzproteins die Position 320 aufweist, eine andere Aminosäure aufweist. Üblicherweise werden solche Austausche auf der DNA-Ebene über Mutationen einzelner Basenpaare durchgeführt (siehe oben). "R320K" bedeutet beispielsweise, dass das Referenzenzym an der Position 320 die Aminosäure Arginin aufweist, während die betrachtete Variante an der hiermit homologisierbaren Position über die Aminosäure Lysin verfügt. "320K" bedeutet, dass jede beliebige, das heißt in der Regel eine natürlicherweise vorgegebene Aminosäure an einer Position, die der Position 320 entspricht, gegen ein Lysin ersetzt ist, welches sich im vorliegenden Molekül eben an dieser Stelle befindet. "R320K, L" bedeutet, dass die Aminosäure Arginin in Position 320 gegen Lysin oder Leucin ersetzt ist. Und "R320X" bedeutet, dass die Aminosäure Arginin in Position 320 gegen eine prinzipiell beliebige andere Aminosäure ersetzt ist.

Grundsätzlich sind die mit der vorliegenden Anmeldung bezeichneten erfindungsgemäßen Aminosäureaustausche nicht darauf beschränkt, dass sie die einzigen Austausche sind, in denen sich die betreffende Variante von dem Wildtypmolekül unterscheidet. Es ist im Stand der Technik bekannt, dass sich die vorteilhaften Eigenschaften einzelner Punktmutationen einander ergänzen können. Somit umfassen Ausführungsformen der vorliegenden Erfindung alle Varianten, die neben anderen Austauschen gegenüber dem Wildtypmolekül auch die erfindungsgemäßen Austausche aufweisen.

Ferner spielt es prinzipiell keine Rolle, in welcher Reihenfolge die betreffenden Aminosäureaustausche vorgenommen worden sind, das heißt ob eine entsprechende Punktmutante erfindungsgemäß weiterentwickelt wird oder zunächst beispielsweise aus einem Wildtypmolekül eine erfindungsgemäße Variante erzeugt wird, die entsprechend anderer im Stand der Technik zu findender Lehren weiterentwickelt wird. Es können auch gleichzeitig in einem Mutageneseansatz mehrere Austausche vorgenommen werden, etwa erfindungsgemäße und andere zusammen.

Es ist erfindungsgemäß bevorzugt, wenn als Enzym mindestens eine Protease enthalten ist. Eine Protease ist ein Enzym, das Peptidbindungen mittels Hydrolyse spaltet. Jedes der Enzyme aus der Klasse E.C. 3.4 fällt erfindungsgemäß darunter (umfassend jede der darunterfallenden dreizehn Unterklassen). Die EC-Nummer entspricht der Enzyme Nomenklatur 1992 der NC-IUBMB, Academic Press, San Diego, California, eingeschlossen der Ergänzungen 1 bis 5, publiziert in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650.

Subtilase benennt eine Untergruppe der Serinproteasen. Die Serinproteasen oder Serinpeptidasen sind eine Untergruppe der Proteasen, die Serin im aktiven Zentrums des Enzyms besitzen, das ein kovalentes Addukt mit dem Substrat bildet. Weiterhin sind die Subtilasen (und die Serineproteasen) dadurch charakterisiert, dass sie neben besagtem Serin mit Histidin und Aspartam zwei weitere Aminosäurereste im aktiven Zentrum aufweisen. Die Subtilasen können in 6 Unterklassen, nämlich die Subtilisin Familie, die Thermitase Familie, die Proteinase K Familie, die Familie der lantibiotischen Peptidasen, die Kexin Familie und die Pyrolysin Familie. Die als Bestandteil der erfindungsgemäßen Waschmittel bevorzugt ausgenommenen oder bevorzugt in reduzierten Mengen enthaltenen Proteasen sind Endopeptidasen (EC 3.4.21).

"Proteaseaktivität" liegt erfindungsgemäß vor, wenn das Enzym proteolytische Aktivität besitzt (EC 3.4). Verschiedenartige Proteaseaktivitäts-Typen sind bekannt: Die drei Haupttypen sind: Trypsin-artig, wobei eine Spaltung des Amidesubstrates nach den Aminosäuren Arg oder Lys bei P1 erfolgt; Chymotrypsin-artig, wobei eine Spaltung nach einer der hydrophoben Aminosäuren bei P1 erfolgt; und Elastase-artig, wobei eine Spaltung des Amidsubstrates nach Ala bei P1 erfolgt.

Die Proteaseaktivität kann nach der in Tenside, Band 7 (1970), S. 125-132 beschriebenen Methode ermittelt werden. Sie wird dementsprechend in PE (Protease-Einheiten) angegeben. Die Proteaseaktivität eines Enzyms lässt sich gemäß gängigen Standardmethoden, wie insbesondere unter Einsatz von BSA als Substrat (Rinderalbumin) und/oder mit der AAPF-Methode.

Überraschenderweise wurde festgestellt, dass eine Protease vom Typ der alkalischen Protease aus Bacillus lentus DSM 5483 oder eine hierzu hinreichend ähnliche Protease (bezogen auf die Sequenzidentität), die mehrere dieser Veränderungen in Kombination aufweist, besonders für den Einsatz in dem erfindungsgemäßen Waschmittel geeignet und darin vorteilhafterweise verbessert stabilisiert wird. Vorteile des Einsatzes dieser Protease ergeben sich somit insbesondere hinsichtlich der Waschleistung und/oder der Stabilität.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. In der vorliegenden Erfindung wurden alle Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Die Konzentration der Protease in dem Waschmittel beträgt von 0,001 bis 0,1 Gew.-%, vorzugsweise von 0,01 bis 0,06 Gew.-%, bezogen auf aktives Protein.

Die erfindungsgemäßen Waschmittel (besonders bevorzugt zusätzlich zur Protease) enthalten bevorzugt als Enzym mindestens ein Enzym, ausgewählt aus α-Amylase, Cellulase, Mannanase, Lipase, Pectatlyase.

Im Allgemeinen können die in einem erfindungsgemäßen Waschmittel enthaltenen Enzyme an Trägerstoffe adsorbiert und/oder in Hüllsubstanzen eingebettet sein, um sie gegen vorzeitige Inaktivierung zu schützen.

Erfindungsgemäße Waschmittel können die erhaltenen Enzyme in jeder nach dem Stand der Technik etablierten Form zugesetzt werden. Hierzu gehören insbesondere die durch Granulation, Extrusion oder Lyophilisierung erhaltenen festen Präparationen, vorteilhafterweise möglichst konzentriert, wasserarm und/oder mit Stabilisatoren versetzt. In einer alternativen Darreichungsform können die Enzyme auch verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind, oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Die Waschmittel enthalten bevorzugt zusätzlich mindestens eine Cellulase. Eine Cellulase ist ein Enzym. Für Cellulasen können synonyme Begriffe verwendet werden, insbesondere Endoglucanase, Endo-1,4-beta-Glucanase, Carboxymethylcellulase, Endo-1,4-beta-D-Glucanase, beta-1,4-Glucanase, beta-1,4-Endoglucanhydrolase, Celludextrinase oder Avicelase. Entscheidend dafür, ob ein Enzym eine Cellulase im Sinne der Erfindung ist, ist deren Fähigkeit zur Hydrolyse von 1,4-ß-D-glucosidischen Bindungen in Cellulose.

Erfindungsgemäß konfektionierbare Cellulasen (Endoglucanasen, EG) umfassen beispielsweise die pilzliche, Endoglucanase(EG)-reiche Cellulase-Präparation beziehungsweise deren Weiterentwicklungen, die von dem Unternehmen Novozymes unter dem Handelsnamen Celluzyme^{®} angeboten wird. Die ebenfalls von dem Unternehmen Novozymes erhältlichen Produkte Endolase^{®} und Carezyme^{®} basieren auf der 50 kD-EG, beziehungsweise der 43 kD-EG aus Humicola insolens DSM 1800. Weitere einsetzbare Handelsprodukte dieses Unternehmens sind Cellusoft^{®}, Renozyme^{®} und Celluclean^{®}. Weiterhin einsetzbar sind beispielsweise Cellulasen, die von dem Unternehmen AB Enzymes, Finnland, unter den Handelsnamen Ecostone^{®} und Biotouch^{®} erhältlich sind, und die zumindest zum Teil auf der 20 kD-EG aus Melanocarpus basieren. Weitere Cellulasen von dem Unternehmen AB Enzymes sind Econase^{®} und Ecopulp^{®}. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax^{®} erhältlich ist. Weitere verwendbare Handelsprodukte des Unternehmens Danisco/Genencor sind "Genencor detergent cellulase L" und IndiAge^{®}Neutra.

Auch durch Punktmutationen erhältliche Varianten dieser Enzyme können erfindungsgemäß eingesetzt werden. Besonders bevorzugte Cellulasen sind Thielavia terrestris Cellulasevarianten, die in der internationalen Offenlegungsschrift WO 98/12307 offenbart sind, Cellulasen aus Melanocarpus, insbesondere Melanocarpus albomyces, die in der internationalen Offenlegungsschrift WO 97/14804 offenbart sind, Cellulasen vom EGIII-Typ aus Trichoderma reesei, die in der europäischen Patentanmeldung EP 1 305 432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Erfindung mit einbezogen.

Erfindungsgemäß besonders bevorzugte Waschmittel sind dadurch gekennzeichnet, dass als zusätzliche Cellulase mindestens eine Cellulase aus *Melanocarpus sp.* oder *Myriococcum sp.* erhältlicher 20K-Cellulase oder solcher, die eine Homologie von über 80% (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) dazu aufweist.

Die aus Melanocarpus sp. oder Myriococcum sp. erhältliche 20K-Cellulase ist aus der internationalen Patentanmeldung WO 97/14804 bekannt. Sie besitzt wie dort beschrieben ein Molekulargewicht von etwa 20 kDa und weist bei 50 °C im pH-Bereich von 4 bis 9 mindestens 80% ihrer maximalen Aktivität auf, wobei noch fast 50% der maximalen Aktivität bei pH 10 erhalten bleiben. Sie kann, wie ebenfalls dort beschrieben, aus Melanocarpus albomyces isoliert und in gentechnisch hergestellten Trichoderma reseei-Transformanten produziert werden. Im Sinne der vorliegenden Erfindung brauchbar sind auch Cellulasen, die eine Homologie von über 80% (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) zur 20K-Cellulase aufweisen.

K20-Cellulase wird vorzugsweise in solchen Mengen verwendet, dass ein erfindungsgemäßes Waschmittel eine cellulolytische Aktivität von 1 NCU/g bis 500 NCU/g (bestimmbar durch die Hydrolyse von 1-gewichtsprozentiger Carboxymethylcellulose bei 50 °C und neutralem pH und Bestimmung der dabei freigesetzten reduzierenden Zucker mittels Dinitrosalicylsäure, wie von M.J.Bailey et al. in Enzyme Microb. Technol. 3: 153 (1981) beschrieben; 1 NCU definiert die Enzymmenge, die reduzierenden Zucker in einer Menge erzeugt, die 1 nmol Glukose pro Sekunde entspricht), insbesondere von 2 NCU/g bis 400 NCU/g und besonders bevorzugt von 6 NCU/g bis 200 NCU/g aufweist. Daneben kann das erfindungsgemäße Waschmittel gegebenenfalls noch weitere Cellulasen enthalten.

Ein erfindungsgemäßes Waschmittel enthält vorzugsweise 0,001 mg bis 0,5 mg, insbesondere 0,02 mg bis 0,3 mg an cellulolytischem Protein pro Gramm des gesamten Waschmittels. Die Proteinkonzentration kann mit Hilfe bekannter Methoden, zum Beispiel dem Bicinchonsäure-Verfahren (BCA-Verfahren, Pierce Chemical Co., Rockford, IL) oder dem Biuret-Verfahren (A.G. Gornall, C.S. Bardawill und M.M. David, J. Biol. Chem. 177, 751-766, 1948) bestimmt werden.

Es ist erfindungsgemäß wiederum besonders bevorzugt, zusätzlich zu mindestens einer ersten Cellulase aus *Melanocarpus sp.* oder *Myriococcum sp.* erhältlicher 20K-Cellulase oder solcher, die eine Homologie von über 80% (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) dazu aufweist mindestens eine weitere von der ersten Cellulase verschiedene zweite Cellulase einzusetzen.

Es ist erfindungsgemäß bevorzugt, wenn die erfindungsgemäßen Waschmittel zusätzlich mindestens eine Lipase enthalten. Erfindungsgemäß bevorzugte Lipase-Enzyme werden ausgewählt aus mindestens einem Enzym der Gruppe, die gebildet wird aus Triacylglycerol-Lipase (E.C. 3.1.1.3) und Lipoprotein-Lipase (E.C. 3.1.1.34) und Monoglycerid-Lipase (E.C. 3.1.1.23).

Ferner ist die in einem erfindungsgemäßen Waschmittel bevorzugt enthaltene Lipase natürlicherweise in einem Mikroorganismus der Art *Thermomyces lanuginosus* oder *Rhizopus oryzae* oder *Mucor javanicus* vorhanden oder von vorgenannten natürlicherweise vorhandenen Lipasen per Mutagenese abgeleitet. Besonders bevorzugt enthalten die erfindungsgemäßen Waschmittel mindestens eine Lipase, die natürlicherweise in einem Mikroorganismus der Art *Thermomyces lanuginosus* vorhanden oder sich von vorgenannten natürlicherweise in *Thermomyces lanuginosus* vorhandenen Lipasen per Mutagenese ableitet. Natürlicherweise vorhanden bedeutet in diesem Zusammenhang, dass die Lipase ein eigenes Enzym des Mikroorganismus ist. Die Lipase kann folglich in dem Mikroorganismus von einer Nukleinsäuresequenz exprimiert werden, die Teil der chromosomalen DNA des Mikroorganismus in seiner Wildtyp-Form ist. Sie bzw. die für sie codierende Nukleinsäuresequenz ist folglich in der Wildtyp-Form des Mikroorganismus vorhanden und/oder kann aus der Wildtyp-Form des Mikroorganismus aus diesem isoliert werden. Im Gegensatz hierzu wäre eine nicht natürlicherweise in dem Mikroorganismus vorhandene Lipase bzw. die für sie codierende Nukleinsäuresequenz mit Hilfe gentechnischer Verfahren in den Mikroorganismus gezielt eingebracht worden, so dass der Mikroorganismus um die Lipase bzw. die für sie codierende Nukleinsäuresequenz bereichert worden wäre. Jedoch kann eine Lipase, die natürlicherweise in einem Mikroorganismus der Art *Thermomyces lanuginosus* oder *Rhizopus oryzae* oder *Mucor javanicus* vorhanden ist, aber durchaus rekombinant von einem anderen Organismus hergestellt worden sein.

Der Pilz *Thermomyces lanuginosus* (auch bekannt unter *Humicola lanuginosa*) zählt zur Klasse der Eurotiomycetes (Unterklasse Eurotiomycetidae), hierin zur Ordnung der Eurotiales und hierin zur Familie Trichocomaceae und der Gattung Thermomyces. Der Pilz *Rhizopus oryzae* zählt zur Klasse der Zygomyceten (Unterklasse Incertae sedis), hierin zur Ordnung Mucorales und hierin wiederum zur Familie Mucoraceae und der Gattung Rhizopus. Der Pilz *Mucor javanicus* zählt ebenfalls zur Klasse der Zygomyceten (Unterklasse Incertae sedis), hierin zur Ordnung Mucorales und hierin wiederum zur Familie Mucoraceae, hierin dann zur Gattung Mucor. Die Bezeichnungen *Thermomyces lanuginosus,* Rhizopus oryzae und Mucor javanicus sind die biologischen Artbezeichnungen innerhalb der jeweiligen Gattung.

Erfindungsgemäß bevorzugte Lipasen sind die von dem Unternehmen Amano Pharmaceuticals unter den Bezeichnungen Lipase M-AP10^{®}, Lipase LE^{®} und Lipase F^{®} (auch Lipase JV^{®}) erhältlichen Lipaseenzyme. Die Lipase F^{®} ist beispielsweise natürlicherweise in Rhizopus oryzae vorhanden. Die Lipase M-AP10^{®} ist beispielsweise natürlicherweise in Mucor javanicus vorhanden.

Waschmittel einer ganz besonders bevorzugten Ausführungsform der Erfindung enthalten mindestens eine Lipase, die ausgewählt wird aus mindestens einem oder mehreren Polypeptiden mit einer Aminosäuresequenz, die zu mindestens 90% (und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) zur Wildtyp Lipase aus dem Stamm DSM 4109 *Thermomyces lanuginosus* identisch ist. Dabei ist es erneut bevorzugt, wenn ausgehend von besagter Wildtyp Lipase aus dem Stamm DSM 4109 zumindest die Aminosäureänderung N233R vorliegt.

Es sind im Rahmen einer weiteren Ausführungsform insbesondere solche Lipasen abgeleitet von der Wildtyp Lipase aus dem Stamm DSM 4109 erfindungsgemäß bevorzugt verwendbar, die ausgewählt werden aus mindestens einem Lipase-Enzym gemäß mindestens einem der Ansprüche 1 bis 13 der Druckschrift WO 00/60063 A1. Auf die Offenbarung Druckschrift WO 00/60063 A1 wird ausdrücklich vollumfänglich Bezug genommen.

Besonders bevorzugt wird in den Waschmitteln der Erfindung mindestens eine Lipase eingesetzt, die abgeleitet ist von der Wildtyp Lipase aus dem Stamm DSM 4109 und in der ausgehend von besagter Wildtyp Lipase mindestens eine Substitution einer elektrisch neutralen oder negativ geladenen Aminosäure durch eine positiv geladene Aminosäure erfolgte. Die Ladung wird in Wasser bei pH 10 bestimmt. Negative Aminosäuren im Sinne der Erfindung sind E, D, Y und C. Positiv geladene Aminosäuren im Sinne der Erfindung sind R, K und H, insbesondere R und K. Neutrale Aminosäure im Sinne der Erfindung sind G, A, V, L, I, P, F, W, S, T, M, N, Q und C, wenn C eine Disulfidbrücke ausbildet.

Im Rahmen dieser Ausführungsform der Erfindung ist es ebenfalls bevorzugt, wenn ausgehend von der Wildtyp Lipase aus dem Stamm DSM 4109 mindestens einen der folgenden Aminosäureaustausche in den Positionen D96L, T213R und/oder N233R, besonders bevorzugt T213R und N233R, vorliegt.

Eine höchst bevorzugte Lipase ist kommerziell unter dem Handelsnamen Lipex^{®} von dem Unternehmen Novozymes (Dänemark) zu beziehen und vorteilhaft in den erfindungsgemäßen Waschmitteln einsetzbar. Besonders bevorzugt ist hierbei die Lipase Lipex^{®} 100 L (ex Novozymes A/S, Dänemark). Bevorzugte Waschmittel sind dadurch gekennzeichnet, dass bezogen auf das Gesamtgewicht des Waschmittels besagtes Lipase-Enzym aus Lipex^{®} 100 L in einer Gesamtmenge von 0,01 bis 1,0 Gew.-%, insbesondere von 0,02 bis 0,1 Gew.-%, enthalten ist.

Die erfindungsgemäßen Waschmittel können als Enzym zusätzlich mindestens eine Mannanase enthalten. Eine in dem erfindungsgemäßen Waschmittels enthaltene Mannanase katalysiert im Rahmen ihrer Mannanase-Aktivität die Hydrolyse von 1,4-beta-D-mannosidischen Bindungen in Mannanen, Galactomannanen, Glucomannanen und Galactoglucomannanen. Besagte erfindungsgemäße Mannanase-Enzyme werden gemäß Enzym Nomenklatur als E.C. 3.2.1.78 klassifiziert.

Die Mannanase-Aktivität eines Polypeptids bzw. Enzyms kann gemäß literaturbekannten Testmethoden bestimmt werden. Dabei wird beispielsweise eine Testlösung in Löcher mit 4 mm Durchmesser einer Agarplatte, enthaltend 0,2 Gew.-% AZGL Galactomannan (carob), i.e. Substrat für das endo-1,4-beta-D-Mannanase Essay, erhältlich unter Katalognummer I-AZGMA der Firma Megazyme (http://www.megazyme.com), eingebracht.

Geeignete erfindungsgemäße Waschmittel enthalten beispielsweise die Mannanase, die unter dem Namen Mannaway^{®} von der Firma Novozymes vermarktet wird.

Mannanase-Enzyme wurden in zahlreichen *Bacillus* Organismen identifiziert:
WO 99/64619 offenbart Beispiele für flüssige, proteasehaltige Waschmittel mit hohem Gesamttensidgehalt von mindestens 20 Gew.-%, die zusätzlich Mannanase-Enzym umfassen.

Bevorzugterweise enthalten die erfindungsgemäßen Waschmittel bezogen auf das Gesamtgewicht des Waschmittels Mannanase in einer Gesamtmenge von 0,01 bis 1,0 Gew.-%, insbesondere von 0,02 bis 0,1 Gew.-%.

Mannanase-Polypeptide aus Stämmen der *Thermoanaerobacter Gruppe,* wie *Caldicellulosiruptor, sind erfindungsgemäß bevorzugt geeignet.* Gleichfalls im Rahmen der Erfindung einsetzbar sind Mannanase-Polypeptide der Pilze *Humicola* oder *Scytalidium,* insbesondere der Species *Humicola insolens* oder *Scytalidium thermophilum.*

Es ist erfindungsgemäß besonders bevorzugt, wenn die erfindungsgemäßen Waschmittel als Mannanase-Enzym mindestens ein Mannanase-Polypeptid aus gram-positiven alkalophilen Stämmen von *Bacillus,* insbesondere ausgewählt aus mindestens einem Vertreter der Gruppe aus *Bacillus subtilis, Bacillus lentus, Bacillus clausii, Bacillus agaradhaerens, Bacillus brevis, Bacillus stearothermophilus, Bacillus alkalophilus, Bacillus amyloliquefaciens, Bacillus coagulans, Bacillus circulans, Bacillus lautus, Bacillus thuringiensis, Bacillus cheniformis, and Bacillus sp., besonders bevorzugt ausgewählt aus mindestens einem Vertreter der Gruppe aus Bacillus sp. 1633, Bacillus sp. AAI12, Bacillus clausii, Bacillus agaradhaerens and Bacillus licheniformis.*

Eine bevorzugte erfindungsgemäße Mannanase wird ausgewählt aus mindestens einem Vertreter aus der Gruppe, die gebildet wird aus
i) Polypeptiden, die eine Aminosäuresequenz umfassen, die mindestens 90% (zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% oder 99,8%) Sequenzidentität zu dem Polypeptid gemäß SEQ ID No.1 (vgl. Sequenzprotokoll aus WO 99/64619), und
ii) Polypeptiden, die ein Fragment von (i) sind.

Dabei ist es wiederum bevorzugt, wenn besagte bevorzugte Mannanase in einer Gesamtmenge von 0,01 bis 1,0 Gew.-%, insbesondere von 0,02 bis 0,1 Gew.-%, jeweils bezogen auf das Gesamtgewicht des Waschmittels, in dem erfindungsgemäßen Waschmittel enthalten ist.

Besonders bevorzugt, enthält das erfindungsgemäße Waschmittel neben der bevorzugten Protease vom Typ der alkalischen Protease aus Bacillus lentus DSM 5483 oder neben der hierzu hinreichend ähnlichen Protease (bezogen auf die Sequenzidentität), die mehrere dieser Veränderungen in Kombination aufweist, zusätzlich mindestens eine α-Amylase.

α-Amylasen (E.C. 3.2.1.1) hydrolysieren als Enzym interne α-1,4-glycosidische Bindungen von Stärke und stärkeähnlichen Polymeren. Diese α-Amylase-Aktivität wird beispielsweise den Anmeldungen WO 97/03160 A1 und GB 1296839 zufolge in KNU (Kilo Novo Units) gemessen. Dabei steht 1 KNU für die Enzymmenge, die 5,25 g Stärke (erhältlich von der Fa. Merck, Darmstadt, Deutschland) pro Stunde bei 37°C, pH 5,6 und in Gegenwart von 0,0043 M Calciumionen hydrolysiert. Eine alternative Aktivitäts-Bestimmungsmethode ist die sogenannte DNS-Methode, die beispielsweise in der Anmeldung WO 02/10356 A2 beschrieben wird. Danach werden die durch das Enzym bei der Hydrolyse von Stärke freigesetzten Oligosaccharide, Disaccharide und Glucoseeinheiten durch Oxidation der reduzierenden Enden mit Dinitrosalicysäure (DNS) nachgewiesen. Die Aktivität wird in µmol reduzierende Zucker (bezogen auf Maltose) pro min und ml erhalten; hierdurch ergeben sich Aktivitätswerte in TAU. Dasselbe Enzym kann über verschiedene Methoden bestimmt werden, wobei die jeweiligen Umrechungsfaktoren je nach Enzym variieren können und somit anhand eines Standards festgelegt werden müssen. Näherungsweise kann man kalkulieren, dass 1 KNU ca. 50 TAU entspricht. Eine weitere Aktivitätsbestimmungsmethode ist die Messung mithilfe des Quick-Start^{®}-Testkits der Fa. Abbott, Abott Park, Illinois, USA.

Ein erfindungsgemäß bevorzugte Einsatzgebiet der erfindungsgemäßen Waschmittel ist die Reinigung von Textilien. Weil Waschmittel für Textilien überwiegend alkalische pH-Werte aufweisen, werden hierfür insbesondere α-Amylasen eingesetzt, die im alkalischen Medium aktiv sind. Solche werden von Mikroorganismen, das heißt Pilzen oder Bakterien, vor allem denen der Gattungen Aspergillus und Bacillus produziert und sekretiert. Ausgehend von diesen natürlichen Enzymen steht weiterhin eine nahezu unüberschaubare Fülle von Varianten zur Verfügung, die über Mutagenese abgeleitet worden sind und je nach Einsatzgebiet spezifische Vorteile aufweisen.

Beispiele hierfür sind die α-Amylasen aus Bacillus licheniformis, aus B. amyloliquefaciens und aus B. stearothermophilus sowie deren für den Einsatz in Waschmitteln verbesserte Weiterentwicklungen. Das Enzym aus B. licheniformis ist von der Firma Novozymes unter dem Namen Termamyl^{®} und von der Firma Genencor unter dem Namen Purastar^{®}ST erhältlich. Weiterentwicklungsprodukte dieser α-Amylase sind von der Firma Novozymes unter den Handelsnamen Duramyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von B. amyloliquefaciens wird von der Firma Novozymes unter dem Namen BAN^{®} vertrieben, und abgeleitete Varianten von der α-Amylase aus B. stearothermophilus unter den Namen BSG^{®} und Novamyl^{®}, ebenfalls von der Firma Novozymes.

Beispiele für α-Amylasen aus anderen Organismen sind die unter den Handelsnamen Fungamyl^{®} von der Firma Novozymes erhältlichen Weiterentwicklungen der α-Amylase aus Aspergillus niger und A. oryzae. Ein weiteres Handelsprodukt ist beispielsweise die Amylase-LT^{®}.

Zum Stand der Technik gehören unter anderem die drei Patentanmeldungen WO 96/23873 A1, WO 00/60060 A2 und WO 01/66712 A2, die von der Fa. Novozymes angemeldet worden sind. WO 96/23873 A1 beschreibt zum Teil mehrere verschiedene Punktmutationen in insgesamt mehr als 30 verschiedenen Positionen in vier verschiedenen Wildtypamylasen und beansprucht solche für alle Amylasen mit mindestens 80% Identität zu einer dieser vier; sie sollen geänderte enzymatische Eigenschaften hinsichtlich der Thermostabilität, der Oxidationsstabilität und der Calciumabhängigkeit aufweisen. Die Anmeldung WO 00/60060 A2 benennt ebenfalls eine Vielzahl an möglichen Aminosäureaustauschen in 10 verschiedenen Positionen an den α-Amylasen aus zwei verschiedenen Mikroorganismen und beansprucht solche für alle Amylasen mit einer Homologie von mindestens 96% Identität zu diesen. WO 01/66712 A2, schließlich, bezeichnet 31 verschiedene, zum Teil mit den zuvor genannten identische Aminosäurepositionen, die in einer der beiden in der Anmeldung WO 00/60060 A2 genannten α-Amylasen mutiert worden sind.

Aus WO 96/23873 A1 geht beispielsweise konkret die Möglichkeit hervor, in den genannten α-Amylasen ein M in Position 9 gemäß der Zählung von AA560 gegen ein L zu ersetzen, in Position 202 M gegen L und die in den Positionen 182 und 183 (beziehungsweise 183 und 184) liegenden Aminosäuren zu deletieren. WO 00/60060 A2 offenbart unter anderem konkret die Aminosäurevariation N195X (das heißt prinzipiell gegen jede andere Aminosäure). WO 01/66712 A2 offenbart unter anderem die Aminosäurevariationen R118K, G186X (darunter insbesondere den hier nicht relevanten Austausch G186R), N299X (darunter insbesondere den hier nicht relevanten Austausch N299A), R320K, E345R und R458K.

Ganz besonders bevorzugt, enthält das erfindungsgemäße Waschmittel neben der bevorzugten Protease vom Typ der alkalischen Protease aus Bacillus lentus DSM 5483 oder eine hierzu hinreichend ähnliche Protease (bezogen auf die Sequenzidentität), die mehrere dieser Veränderungen in Kombination aufweist, zusätzlich mindestens eine α-Amylase, die bei Temperaturen zwischen 10 und 20°C eine höhere Aktivität aufweist, als die Amylase mit dem Handelsnamen "Stainzyme 12 L" der Firma Novozymes.

Erfindungsgemäß bevorzugte Waschmittel enthalten α-Amylase in einer Gesamtmenge von 0,01 bis 1,0 Gew.-%, insbesondere von 0,02 bis 0,1 Gew.-%.

Es ist bevorzugt, dass mindestens ein optischer Aufheller aus den Substanzklassen der Distyrylbiphenyle, der Stilbene, der 4,4'-Diamino-2,2'-stilbendisulfonsäuren, der Cumarine, der Dihydrochinolinone, der 1,3-Diarylpyrazoline, der Naphthalsäureimide, der Benzoxazol-Systeme, der Benzisoxazol-Systeme, der Benzimidazol-Systeme, der durch Heterocyclen substituierten Pyrenderivate und Mischungen daraus ausgewählt wird. Diese Substanzklassen an optischen Aufhellern weisen eine hohe Stabilität, eine hohe Licht- und Sauerstoffbeständigkeit und eine hohe Affinität zu Fasern auf.

Besonders gut und stabil lassen sich die folgenden optischen Aufheller, welche aus der Gruppe bestehend aus Dinatrium-4,4'-bis-(2-morpholino-4-anilino-s-triazin-6-ylamino)stilbendisulfonat, Dinatrium-2,2'-bis-(phenyl-styryl)disulfonat, 4,4'-Bis[(4-anilino-6-[bis(2-hydroxyethyl)amino]-1,3,5-triazin-2-yl)amino]stilben-2,2'-disulfonsäure, Hexanatrium-2,2'-[vinylenbis[(3-sulphonato-4,1-phenylen)imino[6-(diethylamino)-1,3,5-triazin-4,2-diyl]imino]]bis-(benzol-1,4-disulfonat), 2,2'-(2,5-Thiophendiyl)bis[5-1,1-dimethylethyl)-benzoxazol (beispielsweise erhältlich als Tinopal^{®} SFP von BASF SE) und/oder 2,5-Bis(benzoxazol-2-yl)thiophen ausgewählt sind, einarbeiten.

Das Waschmittel kann erfindungsgemäß weiterhin Gerüststoffe umfassen. Als Gerüststoffe sind beispielsweise polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g/mol.

Geeignete Polymere sind insbesondere Polyacrylate, die bevorzugt eine Molekülmasse von 1.000 bis 15.000 g/mol aufweisen. Aufgrund ihrer überlegenen Löslichkeit können aus dieser Gruppe wiederum die kurzkettigen Polyacrylate, die Molmassen von 1.000 bis 10.000 g/mol, und besonders bevorzugt von 1.000 bis 5.000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Als Gerüststoffe, die in dem erfindungsgemäßen Waschmittel enthalten sein können, sind insbesondere auch Silikate, Aluminiumsilikate (insbesondere Zeolithe), Carbonate, Salze organischer Di- und Polycarbonsäuren sowie Mischungen dieser Stoffe zu nennen. Als zusätzliche Gerüststoffe sind insbesondere organische Gerüststoffe geeignet, beispielsweise die in Form ihrer Natriumsalze oder auch als Säuren einsetzbaren Polycarbonsäuren, wobei unter Polycarbonsäuren solche Carbonsäuren verstanden werden, die mehr als eine Säurefunktion tragen. Beispielsweise sind dies Adipinsäure, Bernsteinsäure, Glutarsäure, Äpfelsäure, Weinsäure, Maleinsäure, Fumarsäure, Zuckersäuren, Aminocarbonsäuren, insbesondere Glutaminsäure-N,N-Diessigsäure (GLDA) und Methylglycin-N,N-Diessigsäure (MGDA), sowie Mischungen aus diesen. Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g/mol. Geeignete Polymere sind insbesondere Polyacrylate, die bevorzugt eine Molekülmasse von 1.000 bis 15.000 g/mol aufweisen. Aufgrund ihrer überlegenen Löslichkeit können aus dieser Gruppe wiederum die kurzkettigen Polyacrylate, die Molmassen von 1.000 bis 10.000 g/mol, und besonders bevorzugt von 1.000 bis 5.000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten. In flüssigen Komponenten werden bevorzugt lösliche Gerüststoffe, wie Acrylpolymere mit einer Molmasse von 1.000 bis 5.000 g/mol eingesetzt.

Besonders bevorzugt werden allerdings lösliche Gerüststoffe, wie beispielsweise Zitronensäure, oder Acryl-polymere mit einer Molmassen von 1.000 bis 5.000 g/mol eingesetzt.

Das Waschmittel enthält bevorzugt weiterhin zusätzlich ein oder mehrere nichtwässrige Lösemittel. Geeignete nichtwässrige Lösungsmittel umfassen ein- oder mehrwertige Alkohole oder Glykolether, wie beispielsweise Ethanol, n-Propanol, i-Propanol, Butanolen, Glykol, Propandiol, Butandiol, Methylpropandiol, Glycerin, Glykolen, wie Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3-methoxybutanol, 2,2-dimethyl-4-hydroxymethyl-1,3-dioxolan, Propylen-glykol-t-butylether, Di-n-octylether sowie niedermolekularen Polyalkylenglykolen, wie PEG 400, sowie Mischungen dieser Lösungsmittel.

Vorzugsweise werden die Lösungsmittel ausgewählt aus Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropy-lenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel.

Zusätzlich können die erfindungsgemäßen Waschmittel zusätzlich auch Verbindungen enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Mittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen. Solche sind beispielsweise unter dem Handelsnamen Texcare^{®} kommerziell erhältlich.

Als Antiredepositionsmittel sind insbesondere auf (Co)Polymere auf Basis von Polyethylenimin, Polyvinylacetat und Polyethylenglykol einsetzbar, bevorzugt in Mischungen mit Antiredepositionsmitteln.

Das Waschmittel kann bevorzugt auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf das Waschmittel, eingesetzt werden.

Es ist bevorzugt, dass der Farbübertragungsinhibitor ein Polymer oder Copolymer von cyclischen Aminen wie beispielsweise Vinylpyrrolidon und/oder Vinylimidazol ist. Als Farbübertragungsinhibitor geeignete Polymere umfassen Polyvinylpyrrolidon (PVP), Polyvinylimidazol (PVI), Copolymere von Vinylpyrrolidon und Vinylimidazol (PVP/PVI), Polyvinylpyridin-N-oxid, Poly-N-carboxymethyl-4-vinylpyridiumchlorid, Polyethylenglycolmodifizierte Copolymere von Vinylpyrrolidon und Vinylimidazol sowie Mischungen daraus. Besonders bevorzugt werden Polyvinylpyrrolidon (PVP), Polyvinylimidazol (PVI) oder Copolymere von Vinylpyrrolidon und Vinylimidazol (PVP/PVI) als Farbübertragungsinhibitor eingesetzt. Die eingesetzten Polyvinylpyrrolidone (PVP) besitzen bevorzugt ein mittleres Molekular gewicht von 2.500 bis 400.000 und sind kommerziell von ISP Chemicals als PVP K 15, PVP K 30, PVP K 60 oder PVP K 90 oder von der BASF als Sokalan^{®} HP 50 oder Sokalan^{®} HP 53 erhältlich. Die eingesetzten Copolymere von Vinylpyrrolidon und Vinylimidazol (PVP/PVI) weisen vorzugsweise ein Molekulargewicht im Bereich von 5.000 bis 100.000 g/mol auf. Kommerziell erhältlich ist ein PVP/PVI-Copolymer beispielsweise von der BASF unter der Bezeichnung Sokalan^{®} HP 56. Ein weiterer äußerst bevorzugt einsetzbarer Farbübertragungsinhibitor sind Polyethylenglycolmodifizierte Copolymere von Vinylpyrrolidon und Vinylimidazol, welche beispielsweise unter der Bezeichnung Sokalan^{®} HP 66 von der BASF erhältlich sind.

Das Waschmittel wird insbesondere im Rahmen eines Waschprozesses für Textilien eingesetzt. Die hierin beschriebenen Waschmittel eignen sich einerseits als Waschhilfsmittel, die als Textilvor- und Nachbehandlungsmittel bei der Textilwäsche verwendet werden, also als solche Mittel, mit denen das Wäschestück vor der eigentlichen Wäsche in Kontakt gebracht wird, beispielsweise zum Anlösen hartnäckiger Verschmutzungen.

Das Waschmittel kann in einer für einen Waschgang vorgefertigten Portion (z.B. als wasserlöslichen Mehrkammerpouch) oder ein Mehrkammer-Vorratsgebinde mit eventueller Mischkammer vorliegen.

Eine tensidhaltige Flotte ist im Sinne der Erfindung eine durch Einsatz des Mehrkomponenten-Waschmittels der vorliegenden Erfindung unter Verdünnung mit mindestens einem Lösemittel (bevorzugt Wasser) erhältliche, flüssige Zubereitung für die Behandlung eines Substrats. Als Substrat kommen Gewebe oder Textilien (wie z.B. Kleidung) in Frage. Bevorzugt werden die erfindungsgemäßen Waschmittel zur Bereitstellung einer tensidhaltigen Flotte im Rahmen maschineller Reinigungsverfahren verwendet, wie sie z.B. von einer Waschmaschine für Textilien ausgeführt werden.

Handelt es sich um ein Mehrkomponenten-Waschmittel, so können die weiteren Komponenten des Mehrkomponenten-Waschmittels in jeder nach dem Stand der Technik etablierten und/oder jeder zweckmäßigen Darreichungsform vorliegen. Dazu zählen beispielsweise flüssige, gelförmige oder pastöse Darreichungsformen. Der Behälter ist bevorzugt ein Pouch mit zwei, drei, vier, fünf, sechs, sieben oder acht Kammern, sowohl in Großgebinden als auch portionsweise abgepackt.

Es ist aber erfindungsgemäß ganz besonders bevorzugt, wenn das Mehrkomponenten-Waschmittel ausschließlich flüssige Komponenten umfasst, wobei die flüssigen Komponenten bevorzugt von einer Umhüllung getrennt voneinander konfektioniert sind.

Bei dem Mehrkomponenten-Waschmittel handelt es sich in einer weiteren besonders bevorzugten Ausführungsform um ein Mehrkomponenten-Color-Waschmittel, insbesondere ein flüssiges, also ein Textilwaschmittel für gefärbte Textilien.

Die erfindungsgemäßen Waschmittel enthaltend bevorzugt mindestens ein Alkalisierungsmittel oder dessen Salz in einer Gesamtmenge von 1 bis 20 Gew.-%, bevorzugt von 2 bis 15 Gew.-%.

Die Gesamtmenge des Alkalisierungsmittels und dessen Salz, bzw die Gesamtmenge aller folgenden bevorzugten Vertreter, wird auf Basis der Basenform berechnet, d.h. wenn das Alkalisierungsmittel im erfindungsgemäßen Waschmittel (teilweise) in seiner Salzform vorliegt, wird bei der Mengenberechnung das Gegenion vernachlässigt und für den Salzanteil nur die Basenform ohne das aufgenommene Proton angenommen.

Die Alkalisierungsmittel werden bevorzugt ausgewählt aus (C₂ bis C₆)-Alkanolamin, Natriumhydroxid, Kaliumhydroxid, Natriumcarbonat, Natriumhydrogencarbonat oder Mischungen daraus.

Unter dem Begriff (C₂ bis C₆)-Alkanolamin sind erfindungsgemäß organische Aminverbindungen zu verstehen, die ein Kohlenstoffgerüst aus zwei bis sechs Kohlenstoffatomen besitzen, an das mindestens eine Aminogruppe (bevorzugt genau eine Aminogruppe) und mindestens eine Hydroxygruppe (wiederum bevorzugt genau eine Hydroxygruppe) bindet.

Bei erfindungsgemäß bevorzugten (C₂ bis C₆)-Alkanolaminen handelt es sich um primäre Amine.

Im Rahmen der Erfindung ist es bevorzugt mindestens ein (C₂ bis C₆)-Alkanolamin mit genau einer Aminogruppe einzusetzen. Hierbei handelt es sich wiederum bevorzugt um ein primäres Amin.

Das erfindungsgemäße Waschmittel enthält bevorzugt mindestens ein (C₂ bis C₆)-Alkanolamin ausgewählt unter 2-Aminoethan-1-ol (Monoethanolamin), Tris(2-hydroxyethyl)amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol (insbesondere unter 2-Aminoethan-1-ol, 2-Amino-2-methylpropan-1-ol, 2-Amino-2-methyl-propan-1,3-diol), oder Mischungen davon. Monoethanolamin hat sich als ganz besonders geeignetes (C₂ bis C₆)-Alkanolamin als Alkalisierungsmittel erwiesen.

(C₂ bis C₆)-Alkanolamin oder dessen Salz ist besonders bevorzugt in einer Gesamtmenge von 1 bis 20 Gew.-%, bevorzugt von 2 bis 15 Gew.-%, in den erfindungsgemäßen Waschmitteln enthalten, jeweils bezogen auf die basische Form.

Ein dritter Erfindungsgegenstand ist eine weitere Ausführungsform des erfindungsgemäßen Waschmittels, nämlich ein Mehrkomponenten-Waschmittel, worin mindestens eine Komponente ein Waschmittel (insbesondere ein Flüssigwaschmittel) des zweiten Erfindungsgegenstandes ist und mindestens eine weitere zusätzliche Komponente ausgewählt wird aus mindestens einer Zusammensetzung, ausgewählt aus einer flüssigen Zusammensetzung oder einer pulverförmigen Zusammensetzung oder einem Granulat.

Erfindungsgemäß befindet sich das Mehrkomponenten-Waschmittel bevorzugt in einem Behälter (Pouch) aus wasserlöslichem Material.

Der Behälter für das besagte Mehrkomponenten-Waschmittel umfasst, in verschiedenen Ausführungsformen, mindestens zwei räumlich voneinander getrennte Kammern (Mehrkammerpouch), beispielsweise 2, 3, 4, 5, 6, 7 oder 8 Kammern. Diese Kammern sind derart voneinander getrennt, dass die enthaltenen flüssigen oder flüssigen und festen Komponenten des Waschmittels nicht miteinander in Kontakt treten. Diese Trennung kann beispielsweise durch eine Wand erfolgen, die aus demselben Material besteht wie der Behälter selbst.

Es ist daher erfindungsgemäß besonders bevorzugt, das Mehrkomponenten-Waschmittel, in einem Behälter aus wasserlöslichem Material mit mindestens zwei voneinander getrennten Kammern zu konfektionieren

Wasserlöslich im Sinne der vorliegenden Erfindung ist ein Material, wenn sich bei 20°C 0,1 g des Materials unter Rühren (Rührgeschwindigkeit Magnetrührer 300 rpm, Rührstab: 6,8 cm lang, Durchmesser 10 mm, Becherglas 1000mL niedrige Form der Fa. Schott, Mainz) innerhalb von 600 Sekunden derart in 800 mL Wasser auflöst, dass mit dem bloßen Auge keine einzelnen festförmigen Partikel des Materials mehr sichtbar sind.

Die Wasserlöslichkeit des für die Herstellung von Pouches zur Umhüllung genutzten Materials in Form eines Films kann mit Hilfe eines in einem quadratischen Rahmen (Kantenlänge auf der Innenseite: 20 mm) fixierten quadratischen Films des besagten Materials (Film: 22 x 22 mm mit einer Dicke von 76 µm) nach folgendem Messprotokoll bestimmt werden. Besagter gerahmter Film wird in 800 mL auf 20 °C temperiertes, destilliertes Wasser in einem 1 Liter Becherglas mit kreisförmiger Bodenfläche (Fa. Schott, Mainz, Becherglas 1000 mL, niedrige Form) eingetaucht, so dass die Fläche des eingespannten Films im rechten Winkel zur Bodenfläche des Becherglases angeordnet ist, die Oberkante des Rahmens 1 cm unter der Wasseroberfläche ist und die Unterkante des Rahmens parallel zur Bodenfläche des Becherglases derart ausgerichtet ist, dass die Unterkante des Rahmens entlang des Radius der Bodenfläche des Becherglases verläuft und die Mitte der Unterkante des Rahmens über der Mitte des Radius des Becherglasbodens angeordnet ist. Das Material sollte sich unter Rühren (Rührgeschwindigkeit Magnetrührer 300 rpm, Rührstab: 6,8 cm lang, Durchmesser 10 mm) innerhalb von 600 Sekunden derart auflösen, dass mit dem bloßen Auge keine einzelnen festförmigen Folienpartikel mehr sichtbar sind.

Das wasserlösliche oder wasserdispergierbare Material kann ein Polymer, ein Copolymer oder Mischungen dieser umfassen. Wasserlösliche Polymere im Sinne der Erfindung sind solche Polymere, die bei Raumtemperatur in Wasser zu mehr als 2,5 Gew.-% löslich sind.

Bevorzugte wasserlösliche Materialien umfassen vorzugsweise mindestens anteilsweise wenigstens eine Substanz aus der Gruppe bestehend aus (acetalisierter) Polyvinylalkohol, Polyvinylpyrrolidon, Polyethylenoxid, Gelatine, mit Sulphat, Carbonat und/oder Citrat substituierte Polyvinylalkohole, Polyalkylenoxide, Acrylamide, Celluloseester, Celluloseether, Celluloseamide, Cellulose, Polyvinylacetate, Polycarbonsäuren und deren Salze, Polyaminosäuren oder Peptide, Polyamide, Polyacrylamide, Copolymere von Maleinsäure und Acrylsäure, Copolymere von Acrylamiden und (Meth)Acrylsäure, Polysaccaride, wie beispielsweise Stärke oder Guar-Derivate, Gelatine und unter den INCI Bezeichnungn Polyquaternium 2, Polyquaternium 17, Polyquaternium 18 und Polyquaternium 27 auf. Besonders bevorzugt ist das wasserlösliche Material ein Polivinylalkohol.

In einer Ausführungsform der Erfindung umfasst das wasserlösliche Material Mischungen unterschiedlicher Substanzen. Solche Mischungen ermöglichen die Einstellung der mechanischen Eigenschaften des Behälters und können den Grad der Wasserlöslichkeit beeinflussen.

Das wasserlösliche Material enthält bevorzugt mindestens einen Polyvinylalkohol und/oder mindestens ein Polyvinylalkoholcopolymer. "Polyvinylalkohol" (Kurzzeichen PVAL oder PVA gelegentlich auch PVOH) ist dabei die Bezeichnung für Polymere der allgemeinen Struktur 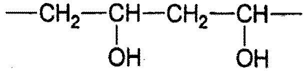 die in geringen Anteilen (ca. 2%) auch Struktureinheiten des Typs 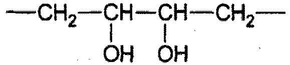 enthalten.

Handelsübliche Polyvinylalkohole, die als weiß-gelbliche Pulver oder Granulate mit Polymerisationsgraden im Bereich von ca. 100 bis 2500 (Molmassen von ca. 4000 bis 100.000 g/mol) angeboten werden, haben Hydrolysegrade von 98 bis 99 Mol-% beziehungsweise 87 bis 89 Mol-%, enthalten also noch einen Restgehalt an Acetyl-Gruppen. Charakterisiert werden die Polyvinylalkohole von Seiten der Hersteller durch Angabe des Polymerisationsgrades des Ausgangspolymeren, des Hydrolysegrades, der Verseifungszahl beziehungsweise der Lösungsviskosität.

Polyvinylalkohole sind abhängig vom Hydrolysegrad löslich in Wasser und wenigen stark polaren organischen Lösungsmitteln (Formamid, Dimethylformamid, Dimethylsulfoxid); von (chlorierten) Kohlenwasserstoffen, Estern, Fetten und Ölen werden sie nicht angegriffen. Polyvinylalkohole werden als toxikologisch unbedenklich eingestuft und sind biologisch zumindest teilweise abbaubar. Die Wasserlöslichkeit kann man durch Nachbehandlung mit Aldehyden (Acetalisierung), durch Komplexierung mit Ni- oder Cu-Salzen oder durch Behandlung mit Dichromaten, Borsäure od. Borax verringern. Die Beschichtungen aus Polyvinylalkohol sind weitgehend undurchdringlich für Gase wie Sauerstoff, Stickstoff, Helium, Wasserstoff, Kohlendioxid, lassen jedoch Wasserdampf hindurchtreten. In bevorzugten Ausführungsformen sind die Polyvinylalkohole im Wesentlichen frei von Ni- und Cu-Salzen und Dichromaten.

Im Rahmen der vorliegenden Erfindung ist es bevorzugt, dass das wasserlösliche Material wenigstens anteilsweise einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% beträgt. In einer bevorzugten Ausführungsform besteht das wasserlösliche Material zu mindestens 20 Gew.-%, besonders bevorzugt zu mindestens 40 Gew.-%, ganz besonders bevorzugt zu mindestens 60 Gew.-% und insbesondere zu mindestens 80 Gew.-% aus einem Polyvinylalkohol, dessen Hydrolysegrad 70 bis 100 Mol-%, vorzugsweise 80 bis 90 Mol%, besonders bevorzugt 81 bis 89 Mol-% und insbesondere 82 bis 88 Mol% beträgt.

Die vorstehend beschriebenen Polyvinylalkohole sind kommerziell breit verfügbar, beispielsweise unter dem Warenzeichen Mowiol^{®} (Clariant). Im Rahmen der vorliegenden Erfindung besonders geeignete Polyvinylalkohole sind beispielsweise Mowiole 3-83, Mowiol^{®} 4-88, Mowiol^{®} 5-88, Mowiol^{®} 8-88 sowie L648, L734, Mowiflex LPTC 221 ex KSE sowie die Compounds der Firma Texas Polymers wie beispielsweise Vinex 2034.

Bevorzugte Polyvinylalkoholcopolymere umfassen neben Vinylalkohol Dicarbonsäuren als weitere Monomere. Geeignete Dicarbonsäure sind Itaconsäure, Malonsäure, Bernsteinsäure und Mischungen daraus, wobei Itaconsäure bevorzugt ist.

Ebenso bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Die Wasserlöslichkeit von Polyvinylalkoholpolymer kann durch Nachbehandlung mit Aldehyden (Acetalisierung) oder Ketonen (Ketalisierung) verändert werden. Als besonders bevorzugt und aufgrund ihrer ausgesprochen guten Kaltwasserlöslichkeit besonders vorteilhaft haben sich hierbei Polyvinylalkohole herausgestellt, die mit den Aldehyd beziehungsweise Ketogruppen von Sacchariden oder Polysacchariden oder Mischungen hiervon acetalisiert beziehungsweise ketalisiert werden.

Weiterhin lässt sich die Wasserlöslichkeit durch Komplexierung mit Ni- oder Cu-Salzen oder durch Behandlung mit Dichromaten, Borsäure, Borax verändern und so gezielt auf gewünschte Werte einstellen. Bevorzugt sind diese nicht enthalten. Folien aus PVAL sind weitgehend undurchdringlich für Gase wie Sauerstoff, Stickstoff, Helium, Wasserstoff, Kohlendioxid, lassen jedoch Wasserdampf hindurchtreten.

Dem als wasserlösliches Material geeigneten Folienmaterial kann neben Polyvinylalkohol zusätzlich Polymere, ausgewählt aus der Gruppe umfassend Acrylsäure-haltige Polymere, Polyacrylamide, Oxazolin-Polymere, Polystyrolsulfonate, Polyurethane, Polyester, Polyether Polymilchsäure, und/oder Mischungen der vorstehenden Polymere, zugesetzt sein.

Geeignete wasserlösliche Folien zum Einsatz als wasserlösliches Material der wasserlöslichen Portion gemäß der Erfindung sind Folien, die unter der Bezeichnung Monosol M8630 oder M8720 von MonoSol LLC vertrieben werden. Andere geeignete Folien umfassen Folien mit der Bezeichnung Solublon^{®} PT, Solublon^{®} KA, Solublon^{®} KC oder Solublon^{®} KL von der Aicello Chemical Europe GmbH oder die Folien VF-HP von Kuraray.

Bevorzugte wasserlösliche Materialien sind dadurch gekennzeichnet, dass sie Hydroxypropylmethylcellulose (HPMC) umfassen, die einen Substitutionsgrad (durchschnittliche Anzahl von Methoxygruppen pro Anhydroglucose-Einheit der Cellulose) von 1,0 bis 2,0, vorzugsweise von 1,4 bis 1,9, und eine molare Substitution (durchschnittliche Anzahl von Hydroxypropoxylgruppen pro Anhydroglucose-Einheit der Cellulose) von 0,1 bis 0,3, vorzugsweise von 0,15 bis 0,25, aufweist.

Polyvinylpyrrolidone, kurz als PVP bezeichnet, werden durch radikalische Polymerisation von 1-Vinylpyrrolidon hergestellt. Handelsübliche PVP haben Molmassen im Bereich von ca. 2.500 bis 750.000 g/mol und werden als weiße, hygroskopische Pulver oder als wässrige Lösungen angeboten.

Polyethylenoxide, kurz PEOX, sind Polyalkylenglykole der allgemeinen Formel

H-[O-CH₂-CH₂]ₙ-OH

die technisch durch basisch katalysierte Polyaddition von Ethylenoxid (Oxiran) in meist geringe Mengen Wasser enthaltenden Systemen mit Ethylenglykol als Startmolekül hergestellt werden. Sie haben üblicherweise Molmassen im Bereich von ca. 200 bis 5.000.000 g/mol, entsprechend Polymerisationsgraden n von ca. 5 bis >100.000. Polyethylenoxide besitzen eine äußerst niedrige Konzentration an reaktiven Hydroxy-Endgruppen und zeigen nur noch schwache Glykol-Eigenschaften.

Gelatine ist ein Polypeptid (Molmasse: ca. 15.000 bis >250.000 g/mol), das vornehmlich durch Hydrolyse des in Haut und Knochen von Tieren enthaltenen Kollagens unter sauren oder alkalischen Bedingungen gewonnen wird. Die Aminosäuren-Zusammensetzung der Gelatine entspricht weitgehend der des Kollagens, aus dem sie gewonnen wurde, und variiert in Abhängigkeit von dessen Provenienz. Die Verwendung von Gelatine als wasserlösliches Hüllmaterial ist insbesondere in der Pharmazie in Form von Hart- oder Weichgelatinekapseln äußerst weit verbreitet. In Form von Folien findet Gelatine wegen ihres im Vergleich zu den vorstehend genannten Polymeren hohen Preises nur geringe Verwendung.

Bevorzugt sind im Rahmen der vorliegenden Erfindung wasserlösliche Materialien, welche ein Polymer aus der Gruppe Stärke und Stärkederivate, Cellulose und Cellulosederivate, insbesondere Methylcellulose und Mischungen hieraus umfassen.

Stärke ist ein Homoglykan, wobei die Glucose-Einheiten α-glykosidisch verknüpft sind. Stärke ist aus zwei Komponenten unterschiedlichen Molekulargewichts (MG) aufgebaut: aus ca. 20 bis 30% geradkettiger Amylose (MG ca. 50.000 bis 150.000 g/mol) und 70 bis 80% verzweigtkettigem Amylopektin (MG ca. 300.000 bis 2.000.000 g/mol). Daneben sind noch geringe Mengen Lipide, Phosphorsäure und Kationen enthalten. Während die Amylose infolge der Bindung in 1,4-Stellung lange, schraubenförmige, verschlungene Ketten mit etwa 300 bis 1.200 Glucose-Molekülen bildet, verzweigt sich die Kette beim Amylopektin nach durchschnittlich 25 Glucose-Bausteinen durch 1,6-Bindung zu einem astähnlichen Gebilde mit etwa 1.500 bis 12.000 Molekülen Glucose. Neben reiner Stärke sind zur Herstellung wasserlöslicher Behälter im Rahmen der vorliegenden Erfindung auch Stärke-Derivate geeignet, die durch polymeranaloge Reaktionen aus Stärke erhältlich sind. Solche chemisch modifizierten Stärken umfassen dabei beispielsweise Produkte aus Veresterungen beziehungsweise Veretherungen, in denen Hydroxy-Wasserstoffatome substituiert wurden. Aber auch Stärken, in denen die Hydroxy-Gruppen gegen funktionelle Gruppen, die nicht über ein Sauerstoffatom gebunden sind, ersetzt wurden, lassen sich als Stärke-Derivate einsetzen. In die Gruppe der Stärke-Derivate fallen beispielsweise Alkalistärken, Carboxymethylstärke (CMS), Stärkeester und -ether sowie Aminostärken.

Reine Cellulose weist die formale Bruttozusammensetzung (C₆H₁₀0₅), auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5.000 Glucose-Einheiten und haben demzufolge durchschnittliche Molmassen von 50.000 bis 500.000 g/mol. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen beziehungsweise Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen.

Das wasserlösliche Material kann weitere Additive aufweisen. Hierbei handelt es sich beispielsweise um Weichmacher, wie beispielsweise Dipropylenglycol, Ethylenglycol oder Diethylenglycol, Wasser oder Aufschlussmittel.

Besonders bevorzugt wird Polyvinylalkohol als wasserlösliches Material eingesetzt. Dieses ist einerseits leicht zu verarbeiten und kostengünstig zu erhalten. Zudem ist es besonders gut in Wasser löslich und ermöglicht so vielfältige Einsatzmöglichkeiten des hergestellten Behälters.

Ein besonders bevorzugtes erfindungsgemäßes Mehrkomponenten-Waschmittel ist dadurch gekennzeichnet, dass das Mehrkomponenten-Waschmittel in Form eines Mehrkammerpouch vorliegt, insbesondere mit einer wasserlöslichen Folie, besonders bevorzugt auf Polyvinylalkohol-Basis.

Darüber hinaus enthalten bevorzugte Mehrkomponenten-Waschmittel vorzugsweise einen Bitterstoff, besonders bevorzugt ist der Bitterstoff in dem Waschmittel und/oder im Behälter des Mehrkomponente-Waschmittels enthalten, insbesondere in der Folie.

Es ist erfindungsgemäß bevorzugt, wenn in dem wasserlöslichen Material zur Erhöhung der Produktsicherheit mindestens ein Bittermittel enthalten ist.

Bevorzugte Bittermittel weisen einen Bitterwert von mindestens 1.000, bevorzugt mindestens 10.000, besonders bevorzugt mindestens 200.000 auf. Zur Bestimmung des Bitterwertes wird das im Europäischen Arzneibuch (5. Ausgabe Grundwerk, Stuttgart 2005, Band 1 Allgemeiner Teil Monografiegruppen, 2.8.15 Bitterwert S. 278) beschriebene standardisierte Verfahren verwendet. Als Vergleich dient eine wässrige Lösung von Chininhydrochlorid, dessen Bitterwert mit 200.000 festgelegt ist. Dies bedeutet, dass 1 Gramm Chininhydrochlorid 200 Liter Wasser bitter macht. Die interindividuellen Geschmacksunterschiede bei der organoleptischen Prüfung der Bitterkeit werden bei diesem Verfahren durch einen Korrekturfaktor ausgeglichen.

Ganz besonders bevorzugte Bittermittel werden ausgewählt aus Denatoniumbenzoat, Glycosiden, Isoprenoiden, Alkaloiden, Aminosäuren und Mischungen daraus, besonders bevorzugt Denatoniumbenzoat.

Glycoside sind organische Verbindungen der allgemeinen Struktur R-O-Z, bei denen ein Alkohol (R-OH) über eine glycosidische Bindung mit einem Zuckerteil (Z) verbunden ist.

Geeignete Glycoside sind beispielsweise Flavonoide wie Quercetin oder Naringin oder Iridoidglycoside wie Aucubin und insbesondere Secoiridoid, wie Amarogentin, Dihydrofoliamentin, Gentiopikrosid, Gentiopikrin, Swertiamarin, Swerosid, Gentioflavosid, Centaurosid, Methiafolin, Harpagosid und Centapikrin, Sailicin oder Kondurangin.

Isoprenoide sind Verbindungen, die sich formal von Isopren ableiten. Beispiele sind insbesondere Terpene und Terpenoide.

Geeignete Isoprenoide umfassen beispielsweise Sequiterpenlactone wie Absinthin, Artabsin, Cnicin, Lactucin, Lactucopikrin oder Salonitenolid, Monoterpen-Ketone (Thujone) wie beispielsweise α-Thujon oder β-Thujon, Tetranortriterpene (Limonoide) wie Desoxylimonen, Desoxylimonensäure, Limonin, Ichangin, Iso-Obacunonsäure, Obacunon, Obacunonsäure, Nomilin oder Nomilinsäure, Terpene wie Marrubin, Prämarrubin, Carnosol, Carnosolsäure oder Quassin.

Alkaloide bezeichnen natürlich vorkommende, chemisch heterogene, meist alkalische, stickstoffhaltige organische Verbindungen des Sekundärstoffwechsels, die auf den tierischen oder menschlichen Organismus wirken.

Geeignete Alkaloide sind beispielsweise Chininhydrochlorid, Chininhydrogensulfat, Chinindihydrochlorid, Chininsulfat, Columbin und Coffein.

Geeignete Aminosäuren umfassen beispielsweise Threonin, Methionin, Phenylalanin, Tryptophan, Arginin, Histidin, Valin und Asparaginsäure.

Besonders bevorzugte Bitterstoffe sind Chininsulfat (Bitterwert = 10.000), Naringin (Bitterwert = 10.000), Saccharoseoctaacetat (Bitterwert = 100.000), Chininhydrochlorid, Denatoniumbenzoat (Bitterwert > 100.000.000) und Mischungen daraus, ganz besonders bevorzugt Denatoniumbenzoat (z.B. erhältlich als Bitrex^{®}).

Das wasserlösliche Material enthält bezogen auf dessen Gesamtgewicht bevorzugt Bittermittel (besonders bevorzugt Denatonium Benzoat) in einer Gesamtmenge von höchstens 1 Gewichtsteil Bitterstoff zu 250 Gewichtsteilen des wasserlöslichen Materials (1 : 250), besonders bevorzugt von höchstens 1 : 500, ganz besonders bevorzugt von höchstens 1 : 1000.

Es ist bevorzugt, das erfindungsgemäße Waschmittel oder erfindungsgemäße Mehrkomponenten-Waschmittel zum Waschen von Textilien, insbesondere zum Entfernen von bleichbaren Anschmutzungen, ganz besonders bevorzugt zur Entfernung von Anschmutzungen, die auf Bestandteilen und Rückständen von Deodorantien, Rost, Beeren, Tee und Rotwein basieren, zu verwenden.

Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung des Waschmittels behandelt wird.

Ein vierter Gegenstand der Erfindung ist deshalb Verfahren zum Waschen von Textilien, umfassend die Schritte,
Zugeben eines Waschmittels des zweiten Erfindungsgegenstandes oder eines Mehrkomponenten-Waschmittels des dritten Erfindungsgegenstandes zu einem Gewebe oder Textil; und

Durchführen einer Waschprozedur, bevorzugt in einer Waschmaschine.

Bevorzugt ist ein Verfahren zur Textilbehandlung umfassend die Verfahrensschritte
(a) Bereitstellen einer wässrigen Flotte durch Mischen von 0,5 Liter bis 40,0 Liter Wasser mit 0,5 bis 100 g eines Waschmittels des zweiten Erfindungsgegenstandes, und
(b) In Kontakt bringen eines Textils mit der gemäß (a) hergestellten wässrigen Flotte.

Es ist bevorzugt, wenn im Schritt (b) das Textil 10 bis 240 Minuten, insbesondere 20 bis 180 Minuten mit der gemäß (a) hergestellten, wässrigen Flotte in Kontakt steht.

Ferner ist es bevorzugt, wenn das Textil nach Schritt (b) gespült und getrocknet wird.

Es ist insbesondere bevorzugt, das Verfahren in einer automatischen Waschmaschine durchzuführen. Dabei wird vor dem Schritt (a) das Textil in die Trommel der Waschmaschine gegeben. Ein Waschmittel des zweiten oder dritten Erfindungsgegenstandes wird in die Einspülkammer der Waschmaschine gefüllt oder gemeinsam mit den Textilien in die Trommel der Waschmaschine gegeben. Dabei ist es bevorzugt, das Mehrkomponenten-Waschmittel des dritten Erfindungsgegenstandes in die Trommel der Waschmaschine zu geben.

Danach werden 0,5 bis 40 Liter Wasser zugefügt und gemischt. Bemerkenswerterweise wird jeweils das Textil nicht durch die erfindungsgemäße Catechol-Metallkomplexverbindung angefärbt. Dies gilt selbst dann, wenn das erfindungsgemäße Waschmittel bzw. Mehrkomponenten-Waschmittel in die Trommel der Waschmaschine dosiert wird und direkt mit dem Textil in Kontakt tritt.

Es ist erfindungsgemäß bevorzugt, wenn zur Herstellung der besagten Lösung 10 bis 110 g, insbesondere 15 bis 100 g des Waschmittels des zweiten Erfindungsgegenstandes mit 5 bis 25 I Wasser, insbesondere mit 10 bis 20 I Wasser, vermischt werden.

In den beschriebenen Verfahren werden in verschiedenen Ausführungsformen der Erfindung Temperaturen von 60°C oder weniger, beispielsweise 50°C oder weniger, eingesetzt. Diese Temperaturangaben beziehen sich auf die im Schritt (b) eingesetzten Temperaturen.

Es kann, wie bereits erwähnt, die Anschmutzung mit dem erfindungsgemäßen Waschmittel dem eigentlichen Waschverfahren vorbehandelt werden und/oder zunächst eine tensidhaltige Flotte als Waschlösung bereitgestellt wird, die das erfindungsgemäße (Mehrkomponenten)-Waschmittel enthält und die anschließend mit dem zu reinigendem Textil in Kontakt gebracht wird.

Alle hierin im Zusammenhang mit den Waschmitteln der Erfindung beschriebenen Ausführungsformen, insbesondere im Hinblick auf die Spezifikation der Inhaltsstoffe, sind gleichermaßen auf die beschriebenen Verfahren und Verwendungen anwendbar und umgekehrt.

### Beispiele

### 1.0 Herstellung und Isolierung der farbigen Komplexe mit N,N'-Dipropyl-2,3-dihydroxyterephthaldiamid (Ligand L)

0,3 mmol N,N'-Dipropyl-2,3-dihydroxyterephthaldiamid (Ligand L) wurden in 10 mL Methanol gelöst. Dazu wurden unter Rühren 0,3 mmol KOH (als 0,5M Lösung in Methanol) gegeben. 0,1 mmol des Metall(III)chlorids bzw. 0,15 mmol des Metall(II)chlorids wurden in 4 mL Methanol gelöst. Diese Lösung wurde anschließend zur ersten Lösung zugegeben. Nach Ablauf einer Rührzeit von einer Stunde, wurde die resultierende Lösung auf 4 mL eingeengt und anschließend 50 mL Diethylether hinzugegeben. Der ausgefällte Metallkomplex wurde durch Filtration isoliert.

Auf diese Weise wurden farbige Pulver der folgenden Metallkomplexe Fe(III)L₃ (rot), Ce(III)L₃ (violett) und Mn(II)L₂ (grün), hergestellt.

Aus einer Spatelspitze eines Farbstoffes wurden unter pH-Wert Einstellung mit NaOH jeweils eine Lösung pro Farbstoff und pH-Wert bei pH 8, pH 9,5 und pH 10 hergestellt. Eine Anhebung des pH Wertes der jeweiligen Lösungen führte zu keiner sichtbaren Farbänderung.

Von den Komplexen Fe(III)L₃ (rot), Ce(III)L₃ (violett) und Mn(II)L₂ (grün) wurden UV/VIS-Spektren in wässriger Lösung bei pH 8 (NaOH) und 20°C aufgenommen.

**Tabelle 1: Maxima im UV/VIS-Spektrum bei einer Wellenlänge zwischen 400 und 800 nm**

| Catechol-Metallkomplex | Maximum (Wellenlänge in nm) |
|---|---|
| Fe(III)L₃ | 444 (Schulter 495) |
| Ce(III)L₃ | 531 |
| Mn(II)L₂ | 626 |

### 2.0 Herstellung erfindungsgemäßer Waschmittel

Es wurden folgende erfindungsgemäße Waschmittel gemäß Tabelle 2 unter Rühren hergestellt.

**Tabelle 2: Flüssige Waschmittel**

| | F1 [Gew.-%] | F2 [Gew.-%] | F3 [Gew.-%] | F4 [Gew.-%] | F5 [Gew.-%] | F6 [Gew.-%] |
|---|---|---|---|---|---|---|
| C₁₁₋₁₃-Alkylbenzolsulfonsäure | 22,0 | 26,0 | 22,0 | 26,0 | 9,0 | 3,0 |
| (C₁₂₋₁₄)-Fettalkoholethersulfat mit 2 Einheiten Ethylenoxid | - | - | - | - | 9,0 | 4,6 |
| an 2-Position verzweigter C₁₃₋₁₅-Alkylalkohol ethoxyliert mit 8 Mol Ethylenoxid | 24,0 | 27,0 | 24,0 | 27,0 | 6,0 | - |
| Fettalkoholether ethoxyliert mit 7 Mol Ethylenoxid | - | - | - | - | - | 3,7 |
| Glyzerin | 10,5 | 9,0 | 10,5 | 9,0 | - | - |
| 2-Aminoethanol | 6,0 | 6,8 | 6,0 | 6,8 | - | - |
| Natriumhydroxid | - | - | - | - | 4,0 | 0,6 |
| ethoxyliertes Polyethylenimin | 6,0 | 5,0 | 6,0 | 5,0 | - | - |
| C₁₂₋₁₈-Fettsäure | 7,5 | 7,5 | 7,5 | 7,5 | 1,0 | 1,3 |
| Diethylentriamin-N,N,N',N',N"-penta(methylenphosphonsäure), Heptanatriumsalz | 0,7 | 0,6 | 0,7 | 0,6 | 3,0 | 0,2 |
| Zitronensäure | - | - | - | - | ad pH 8,5 | ad pH 8,5 |
| Borsäure | - | - | - | - | 1,0 | 0,5 |
| 1,2-Propylenglykol | 8,2 | 4,5 | 8,2 | 4,5 | 2,0 | 0,5 |
| Ethanol | 3,0 | 4,0 | 3,0 | 4,0 | 1,0 | 0,2 |
| Natriumbisulfit | - | 0,1 | - | 0,1 | - | - |
| Denatoniumbenzoat | 0,001 | 0,001 | 0,001 | 0,001 | 0,001 | 0,001 |
| Soil-Release Polymer aus Ethylenterephthalat und Polyethylenoxid-terephthalat | 1,4 | 1,0 | 1,4 | 1,0 | 0,5 | - |
| Sokalan HP 56 | - | 0,1 | - | 0,1 | 0,2 | - |
| Optischer Aufheller | 0,6 | - | 0,6 | - | | |
| Parfüm | 1,7 | 1,7 | 1,7 | 1,7 | 2,6 | 1,0 |
| Protease, Amylase, Lipase, Cellulase | 0,8 | 0,8 | 0,8 | 0,8 | 1,0 | 1,0 |
| Fe(III)L₃ | 0,015 | 0,015 | - | - | 0,015 | - |
| Mn(II)L₂ | - | - | 0,05 | 0,05 | - | 0,05 |
| Wasser | ad 100 | ad 100 | ad 100 | ad 100 | ad 100 | ad 100 |

Die Rezepturen F1 bis F6 wurden bei 5°C, 21°C und 40°C jeweils für 4 Wochen gelagert. Die Zusammensetzungen waren lagerstabil und wiesen im Vergleich zum Ausgangsprodukt vor der Lagerung keine mit bloßem Auge erkennbare Verfärbung auf.

### 3.0 Waschversuche

Weiterhin wurden mit den Waschmitteln F1 und F3 der Tabelle 2 jeweils Waschversuche nach den folgenden Testbedingungen durchgeführt.

Weiße Gewebe (WFK 10A, WFK 20A und WFK 30A) wurden in einer Miele Waschmachine Typ 318 bei 16°d und 40°C im Kurzprogramm zweimal in Folge mit 35 g des Pulverwaschmittels des Handelsproduktes "Weißer Riese Universal" gewaschen. Anschließend wurde ein Waschgang ohne Waschmittel durchgeführt. Gewebe WFK 10 A wird von "wfk Testgewebe GmbH" als "Standard Cotton" (Standard Baumwolle), WFK 20A als "Polyester/Cotton (65 %/35 %)" (Polyester/Baumwolle 65 %/35%) und WFK 30A als "Polyester" bezeichnet.

Die wie zuvor beschriebenen, vorgewaschenen, weißen Gewebe (WFK 10A, WFK 20A und WFK 30A) wurden neben 23 weißen Handtüchern, 5 weißen Frotteetüchern und 6 weißen T-Shirts (Waschladung insgesamt 3 kg) mit 25 g der entsprechenden erfindungsgemäßen Rezeptur der Tabelle 2 in einer Miele Waschmachine Typ 318 bei 16°d und 40°C im Hauptwaschgang 60 Minuten lang gewaschen, anschließend gespült und hängend getrocknet. Diese Prozedur wurde 3 Mal hintereinander durchgeführt.

Die weißen Gewebe wiesen keine Verfärbungen sowie keine fleckigen Einfärbungen auf. Das beweist, dass der erfindungsgemäß eingesetzte Catechol-Metallkomplex auch bei wiederholtem Waschvorgang nicht das Textil anfärbt.

## Patentansprüche

1. Waschmittel, insbesondere Flüssigwaschmittel, enthaltend jeweils bezogen auf das Gesamtgewicht des Waschmittels
- in einer Gesamtmenge von 0,001 bis 10,0 Gew.-%, bevorzugt 0,01 bis 3,0 Gew.-%, mindestens eine Catechol-Metallkomplexverbindung der Formel (I) 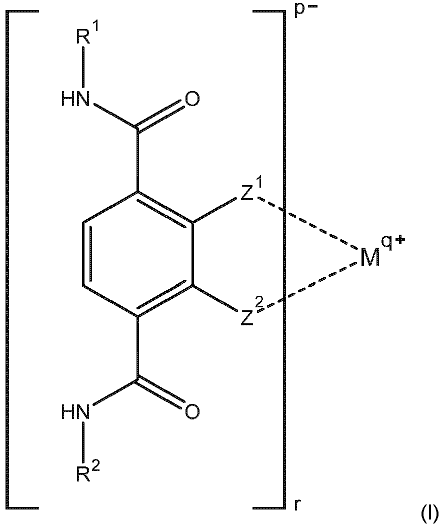 wobei
R¹ und R² unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X⁻, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen,
Z¹ und Z² unabhängig voneinander für OH oder O⁻ stehen,
M steht für ein Metallkation eines Übergangsmetalls oder Lanthanoids, (insbesondere für ein Metallkation aus Ti, Zr, Hf, V, Nb, Ta, Cr. Mo, W, Mn, Fe, Ru, Co, Ni, Cu, Zn, Ce oder Sm),
q als Ladungszahl des Metallkations M für eine Zahl 2, 3 oder 4 steht,
p als Ladungszahl des Catecholliganden für eine Zahl 0, 1 oder 2 steht,
r für eine Zahl 1, 2, 3 oder 4 steht,
und
- mindestens ein Tensid, bevorzugt in einer Gesamtmenge von 2 bis 70 Gew.-%, insbesondere von 10 bis 65 Gew.-%, besonders bevorzugt von 15 bis 60 Gew.-%.

2. Waschmittel nach Anspruch 1, **dadurch gekennzeichnet, dass** in Formel (I) die Reste R¹ und R² unabhängig voneinander für eine Alkylgruppe, eine Alkoxyalkylgruppe, eine Hydroxyalkylgruppe, eine Hydroxyalkyloxyalkyl-Gruppe, (N-Hydroxyethyl)-aminoethyl, (N-Methoxyethyl)-aminoethyl oder (N-Ethoxyethyl)-aminoethyl, oder eine aromatische Gruppe stehen (bevorzugt für Methyl, ethyl, n-Propyl, Isopropyl, n-Butyl, sec-Butyl, tert-Butyl, iso-Butyl, n-pentyl, iso-Pentyl, Neopentyl, Hexyl, 2-Hydroxyethyl, 2-Hydroxypropyl, 3-Hydroxypropyl, 2-Methoxyethyl, 2-Ethoxyethyl, 3-Methoxypropyl, 3-Ethoxypropyl, (N-Hydroxyethyl)-aminoethyl, (N-Methoxyethyl)-aminoethyl oder (N-Ethoxyethyl)-aminoethyl oder Phenyl).

3. Waschmittel nach einem der Ansprüche 1 oder 2, **dadurch gekennzeichnet, dass** in Formel (I) die Reste R¹ und R² gleich sind.

4. Waschmittel nach einem der Ansprüche 1 bis 3, **dadurch gekennzeichnet, dass** in Formel (I) M ausgewählt ist aus Fe, Mn, Cr, Ni, Co, Ce, Cu oder Hydraten dieser Metallionen.

5. Waschmittel nach einem der Ansprüche 1 bis 4, wobei das Waschmittel einen pH-Wert bei 20°C von 6 bis 11,5, bevorzugt 6,5 bis 9,5 , stärker bevorzugt 7,0 bis 9,0 , aufweist.

6. Waschmittel nach einem der Ansprüche 1 bis 5, **dadurch gekennzeichnet, dass** mindestens ein anionisches Tensid und/oder mindestens ein nichtionisches Tensid enthalten ist.

7. Waschmittel nach einem der Ansprüche 1 bis 6, **dadurch gekennzeichnet, dass** die Catechol-Metallkomplexverbindung der Formel (I) Licht in einer Wellenlänge von 400 bis 800 nm absorbiert, gemessen mittels Zweistrahl-Spektralphotometer bei einer Konzentration des Komplexes von 10⁻⁵ mol/L in Wasser bei 20°C, einem pH-Wert von 8 und einer Schichtdicke von 1 cm.

8. Waschmittel nach einem der Ansprüche 1 bis 7, **dadurch gekennzeichnet, dass** es zusätzlich mindestens eine freie Catecholverbindung der Formel (II) oder deren Salz enthält 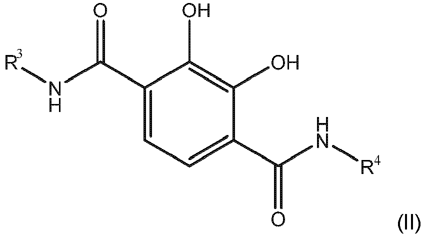 wobei
R³ und R⁴ unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X⁻, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen,
mit der Maßgabe, dass die Catecholverbindung der Formel (II) und deren Salz von Verbindungen der Formel (I) verschieden sind.

9. Waschmittel nach Anspruch 8, **dadurch gekennzeichnet, dass** es die freie Catecholverbindung der Formel (II) in einer Gesamtmenge von 0,01 bis 10,0 Gew.-%, bevorzugt von 0,1 bis 5,0 Gew.-%, enthält.

10. Mehrkomponenten-Waschmittel, **dadurch gekennzeichnet, dass** mindestens eine Komponente ein Waschmittel, insbesondere ein Flüssigwaschmittel nach einem der Ansprüche 1 bis 9 ist und mindestens eine weitere zusätzliche Komponente ausgewählt wird aus mindestens einer Zusammensetzung, ausgewählt aus einer flüssigen Zusammensetzung oder einer pulverförmigen Zusammensetzung oder einem Granulat.

11. Mehrkomponenten-Waschmittel nach Anspruch 10, **dadurch gekennzeichnet, dass** das Mehrkomponenten-Waschmittel in Form eines Mehrkammerpouch vorliegt, insbesondere mit einer wasserlöslichen Folie, besonders bevorzugt auf Polyvinylalkohol-Basis.

12. Mehrkomponenten-Waschmittel nach einem der Ansprüche 10 oder 11, **dadurch gekennzeichnet, dass** das Mehrkomponenten-Waschmittel einen Bitterstoff enthält, bevorzugt ist der Bitterstoff in dem Waschmittel und/oder im Behälter des Mehrkomponente-Waschmittels enthalten, insbesondere in der Folie.

13. Verwendung des Waschmittels nach einem der Ansprüche 1 bis 9 oder des Mehrkomponenten-Waschmittels nach einem der Ansprüche 10 bis 12, zum Entfernen von bleichbaren Anschmutzungen.

14. Verfahren zum Waschen von Textilien, umfassend die Schritte,
Zugeben eines Waschmittels nach einem der Ansprüche 1 bis 9 oder eines Mehrkomponenten-Waschmittels nach einem der Ansprüche 10 bis 12 zu einem Gewebe oder Textil; und
Durchführen einer Waschprozedur, bevorzugt in einer Waschmaschine.

## Claims

1. Detergent, in particular liquid detergent, comprising, in each case based on the total weight of the detergent
- in a total amount of 0.001 to 10.0% by weight, preferably 0.01 to 3.0% by weight, of at least one catechol metal complex compound represented by formula (I) 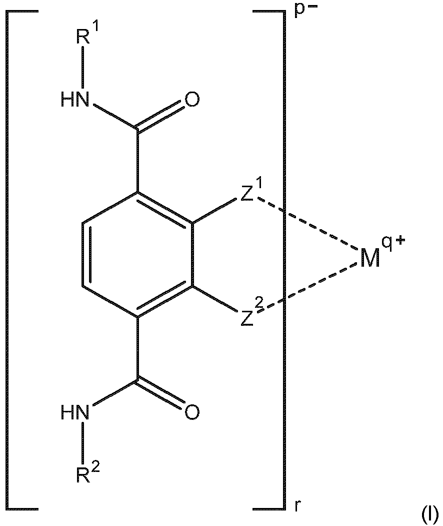 where
R¹ and R² independently of one another represent a hydrocarbon radical having 1 to 20 carbon atoms which is optionally substituted by at least one radical selected from hydroxyl, (C₁ -C₄ )-alkoxy, (C₁ -C₄ )-alkoxy(CH₂ CH₂ O)ₙ -,-NR'R" or -N⁺R'R"R‴ X⁻, where n = 1 to 10, R', R" and R‴ independently of one another represent H or a linear or branched aliphatic hydrocarbon radical having 1 to 3, preferably 1 to 2 carbon atoms, and X⁻ represents an anion,
Z¹ and Z² independently stand for OH or O⁻ ,
M stands for a metal cation of a transition metal or lanthanide, (in particular for a metal cation of Ti, Zr, Hf, V, Nb, Ta, Cr. Mo, W, Mn, Fe, Ru, Co, Ni, Cu, Zn, Ce or Sm),
Q as the charge number of the metal cation M represents a number 2, 3 or 4,
p as the charge number of the catechol ligand represents a number 0, 1 or 2,
r stands for a number 1, 2, 3 or 4,
and
- at least one surfactant, preferably in a total amount of from 2 to 70% by weight, in particular from 10 to 65% by weight, more preferably from 15 to 60% by weight.

2. Detergent according to claim 1, **characterized in that** in formula (I) the radicals R¹ and R² independently of one another represent an alkyl group, an alkoxyalkyl group, a hydroxyalkyl group, a hydroxyalkyloxyalkyl group, (N-hydroxyethyl)-aminoethyl, (N-methoxyethyl)-aminoethyl or (N-ethoxyethyl)-aminoethyl, or an aromatic group (preferably methyl, ethyl, n-propyl, isopropyl, n-butyl, sec-butyl, tert-butyl, iso-butyl, n-pentyl, iso-pentyl, neopentyl, hexyl, 2-hydroxyethyl, 2-hydroxypropyl, 3-hydroxypropyl, 2-methoxyethyl, 2-ethoxyethyl, 3-methoxypropyl, 3-ethoxypropyl, (N-hydroxyethyl)aminoethyl, (N-methoxyethyl)aminoethyl or (N-ethoxyethyl)aminoethyl or phenyl).

3. Detergent according to one of claims 1 or 2, **characterized in that** in formula (I) the radicals R¹ and R² are identical.

4. Detergent according to any one of claims 1 to 3, **characterized in that** in formula (I) M is selected from Fe, Mn, Cr, Ni, Co, Ce, Cu or hydrates of these metal ions.

5. Detergent according to any one of claims 1 to 4, wherein the detergent has a pH at 20°C of 6 to 11.5, preferably 6.5 to 9.5 , more preferably 7.0 to 9.0 .

6. Detergent according to any one of claims 1 to 5, **characterized in that** at least one anionic surfactant and/or at least one nonionic surfactant is present.

7. Detergent according to any one of claims 1 to 6, **characterized in that** the catechol metal complex compound of formula (I) absorbs light in a wavelength of 400 to 800 nm, measured by a double-beam spectrophotometer at a concentration of the complex of 10⁻⁵ mol/L in water at 20°C, a pH of 8 and a layer thickness of 1 cm.

8. Detergent according to any one of claims 1 to 7, **characterized in that** it additionally contains at least one free catechol compound of the formula (II) or its salt 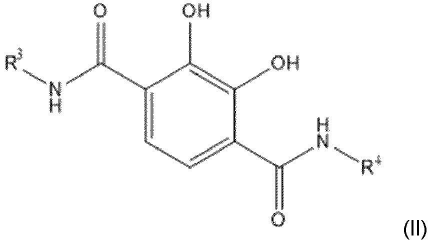 where
R³ and R⁴ independently of one another represent a hydrocarbon radical having 1 to 20 carbon atoms which is optionally substituted by at least one radical selected from hydroxyl, (C₁ -C₄ )-alkoxy, (C₁ -C₄ )-alkoxy(CH₂ CH₂ O)ₙ -, NR'R" or
-N⁺R'R"R‴ X, where n = 1 to 10, R', R" und R‴ independently of one another represent H or a linear or branched aliphatic hydrocarbon radical having 1 to 3, preferably 1 to 2 carbon atoms, and X⁻ represents an anion,
provided that the catechol compound of formula (II) and its salt are different from compounds of formula (I).

9. Detergent according to claim 8, **characterized in that** it contains the free catechol compound of formula (II) in a total amount of from 0.01 to 10.0% by weight, preferably from 0.1 to 5.0% by weight.

10. Multicomponent detergent, **characterized in that** at least one component is a detergent, in particular a liquid detergent according to any one of claims 1 to 9, and at least one further additional component is selected from at least one composition selected from a liquid composition or a powder composition or a granule.

11. Multicomponent detergent according to claim 10, **characterized in that** the multicomponent detergent is in the form of a multicompartment pouch, in particular with a water-soluble film, particularly preferably based on polyvinyl alcohol.

12. Multicomponent detergent according to one of claims 10 or 11, **characterized in that** the multicomponent detergent contains a bittering agent, preferably the bittering agent is contained in the detergent and/or in the container of the multicomponent detergent, in particular in the film.

13. Use of the detergent according to any one of claims 1 to 9 or the multicomponent detergent according to any one of claims 10 to 12, for removing bleachable soils.

14. Method for washing textiles, comprising the steps,
Adding a detergent according to any one of claims 1 to 9 or a multicomponent detergent according to any one of claims 10 to 12 to a fabric or textile; and
Performing a washing procedure, preferably in a washing machine.

## Revendications

1. Détergent, en particulier détergent liquide, contenant par rapport au poids total du détergent
- en une quantité totale de 0,001 à 10,0 % en poids, de préférence de 0,01 à 3,0 % en poids, au moins un composé complexe métallique de catéchol de formule (I) 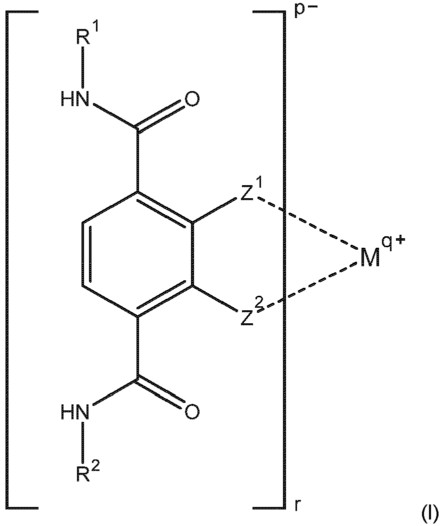 où
R¹ et R² représentent, indépendamment l'un de l'autre, un radical hydrocarboné ayant de 1 à 20 atomes de carbone, qui est éventuellement substitué par au moins un radical choisi parmi les radicaux hydroxy, (C₁ -C₄ )-alcoxy, (C₁ -C₄ )-alcoxy(CH₂ CH₂ O)ₙ -, - NR'R" ou - N⁺R'R"R‴ X , où n = 1 à 10, R', R" et R"' représentent indépendamment les uns des autres H ou un radical hydrocarboné aliphatique linéaire ou ramifié ayant de 1 à 3, de préférence de 1 à 2 atomes de carbone et X⁻ représente un anion,
Z¹ et Z² représentent indépendamment l'un de l'autre OH ou O⁻ ,
M représente un cation métallique d'un métal de transition ou d'un lanthanoïde, (en particulier un cation métallique de Ti, Zr, Hf, V, Nb, Ta, Cr. Mo, W, Mn, Fe, Ru, Co, Ni, Cu, Zn, Ce ou Sm),
Q est le nombre de charges du cation métallique M et est égal à 2, 3 ou 4,
p , en tant que nombre de charge du ligand du catéchol, représente un nombre 0, 1 ou 2,
r représente un nombre 1, 2, 3 ou 4,
et
- au moins un agent tensioactif, de préférence en une quantité totale de 2 à 70 % en poids, en particulier de 10 à 65 % en poids, de manière particulièrement préférée de 15 à 60 % en poids.

2. Détergent selon la revendication 1, **caractérisé en ce que** dans la formule (I), les radicaux R¹ et R² représentent indépendamment l'un de l'autre un groupe alkyle, un groupe alcoxyalkyle, un groupe hydroxyalkyle, un groupe hydroxyalkyloxyalkyle, le (N-hydroxyéthyl)-aminoéthyle, le (N-méthoxyéthyl)-aminoéthyle ou le (N-éthoxyéthyl)-aminoéthyle, ou un groupe aromatique (de préférence le méthyle, éthyle, n-propyle, isopropyle, n-butyle, sec-butyle, tert-butyle, isobutyle, n-pentyle, isopentyle, néopentyle, hexyle, 2-hydroxyéthyle, 2-hydroxypropyle, 3-hydroxypropyle, 2-méthoxyéthyle, 2-éthoxyéthyle, 3-méthoxypropyle, 3-éthoxypropyle, (N-hydroxyéthyl)-aminoéthyle, (N-méthoxyéthyl)-aminoéthyle ou (N-éthoxyéthyl)-aminoéthyle ou phényle).

3. Détergent selon l'une des revendications 1 ou 2, **caractérisé en ce que** dans la formule (I), les radicaux R¹ et R² sont identiques.

4. Détergent selon l'une quelconque des revendications 1 à 3, **caractérisée en ce que** dans la formule (I), M est choisi parmi Fe, Mn, Cr, Ni, Co, Ce, Cu ou des hydrates de ces ions métalliques.

5. Détergent selon l'une quelconque des revendications 1 à 4, dans laquelle la détergent présente un pH à 20°C de 6 à 11,5, de préférence de 6,5 à 9,5 , plus préférentiellement de 7,0 à 9,0 .

6. Détergent selon l'une des revendications 1 à 5, **caractérisée en ce qu'**elle contient au moins un agent tensioactif anionique et/ou au moins un agent tensioactif non ionique.

7. Détergent selon l'une des revendications 1 à 6, **caractérisé en ce que** le composé complexe métallique de catéchol de formule (I) absorbe la lumière dans une longueur d'onde de 400 à 800 nm, mesurée au moyen d'un spectrophotomètre à deux faisceaux à une concentration du complexe de 10⁻⁵ mol/L dans l'eau à 20°C, à un pH de 8 et à une épaisseur de couche de 1 cm.

8. Détergent selon l'une des revendications 1 à 7, **caractérisée en ce qu'**elle contient en outre au moins un composé catéchol libre de formule (II) ou son sel 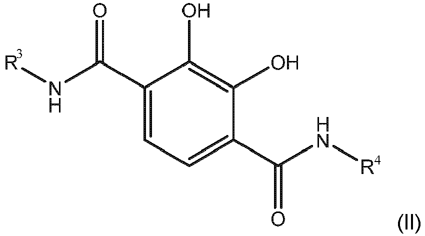 où
R³ et R⁴ représentent, indépendamment l'un de l'autre, un radical hydrocarboné ayant de 1 à 20 atomes de carbone, qui est éventuellement substitué par au moins un radical choisi parmi les radicaux hydroxy, (C₁ -C₄ )-alcoxy, (C₁ -C₄ )-alcoxy(CH₂ CH₂ O)ₙ -, - NR'R" ou
- N⁺R'R"R‴ X⁻, où n = 1 à 10, R' , R" et R‴ représentent indépendamment les uns des autres H ou un radical hydrocarboné aliphatique linéaire ou ramifié ayant de 1 à 3, de préférence de 1 à 2 atomes de carbone et X⁻ représente un anion,
à condition que le composé catéchol de formule (II) et son sel soient différents des composés de formule (I).

9. Détergent selon la revendication 8, **caractérisé en ce qu'**il contient le composé catéchol libre de formule (II) en une quantité totale de 0,01 à 10,0 % en poids, de préférence de 0,1 à 5,0 % en poids.

10. Détergent à plusieurs composants, **caractérisé en ce qu'**au moins un composant est un détergent, en particulier un détergent liquide selon l'une des revendications 1 à 9 et au moins un autre composant supplémentaire est choisi parmi au moins une composition choisie parmi une composition liquide ou une composition en poudre ou un granulé.

11. Détergent à plusieurs composants selon la revendication 10, **caractérisé en ce que** le détergent à plusieurs composants se présente sous la forme d'une pochette à plusieurs compartiments, en particulier avec un film soluble dans l'eau, de manière particulièrement préférée à base d'alcool polyvinylique.

12. Détergent à plusieurs composants selon l'une des revendications 10 ou 11, **caractérisé en ce que** le détergent à plusieurs composants contient une substance amère, de préférence la substance amère est contenue dans le détergent et/ou dans le récipient du détergent à plusieurs composants, en particulier dans le film.

13. Utilisation du détergent selon l'une des revendications 1 à 9 ou du détergent à plusieurs composants selon l'une des revendications 10 à 12, pour éliminer des salissures pouvant être blanchies.

14. Procédé de lavage de textiles, comprenant les étapes consistant à
ajouter à un tissu ou à un textile un détergent selon l'une quelconque des revendications 1 à 9 ou un détergent à plusieurs composants selon l'une quelconque des revendications 10 à 12 ; et
Exécution d'une procédure de lavage, de préférence dans une machine à laver.
